(19) Europäisches Patentamt
European Patent Office
Office européen des brevets

(11) Publication number: **0 079 522**
**B1**

# (12) EUROPEAN PATENT SPECIFICATION

(45) Date of publication of patent specification: **07.05.86**

(51) Int. Cl.⁴: **C 07 K 5/06,** C 07 K 1/06, A 61 K 37/02

(21) Application number: **82110115.1**

(22) Date of filing: **03.11.82**

## (54) N-Carboxymethyl(amidino)lysyl-proline antihypertensive agents.

(30) Priority: **09.11.81 US 319341**

(43) Date of publication of application:
**25.05.83 Bulletin 83/21**

(45) Publication of the grant of the patent:
**07.05.86 Bulletin 86/19**

(84) Designated Contracting States:
**CH DE FR GB IT LI NL**

(56) References cited:
**EP-A-0 012 401**
**EP-A-0 046 953**
**US-A-4 296 110**

(73) Proprietor: **MERCK & CO. INC.**
**126, East Lincoln Avenue P.O. Box 2000**
**Rahway New Jersey 07065 (US)**

(72) Inventor: **Patchett, Arthur A.**
**1090 Minisink Way**
**Westfield New Jersey 07090 (US)**
Inventor: **Wu, Mu Tsu**
**35 Lance Drive**
**Clark New Jersey 07066 (US)**

(74) Representative: **Abitz, Walter, Dr.-Ing. et al**
**Abitz, Morf, Gritschneder, Freiherr von**
**Wittgenstein Postfach 86 01 09**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 079 522 B1

**Description**

BACKGROUND OF THE INVENTION

1. *Field of the Invention*

The present invention is concerned with novel N-carboxymethyl(amidino)lysyl-proline compounds which are effective inhibitors of angiotensin I converting enzyme. These novel compounds are, consequently, combined with pharmaceutically acceptable carriers to form pharmaceutical compositions of the present invention and are used in a method of treating hypertension.

Angiotensin II, a powerful vasoconstrictor hormonal peptide, is formed from the inactive angiotensin I by the action of angiotensin-converting enzyme. Recently, potent inhibitors of angiotensin-converting enzyme have been reported which are capable of lowering the blood pressure in hypertensive patients. The novel N-carboxymethyl(amidino)lysyl-proline compounds of the present invention are also potent inhibitors of angiotensin-converting enzyme.

2. *Brief Description of the Prior Art*

U.S. Patents 4,113,715; 4,129,571; and 4,154,960 disclose substituted acyl derivatives of amino acids which are useful as angiotension converting enzyme inhibitors. More specifically, these compounds are mercapto substituted acyl amino acids and derivatives thereof including the clinically effective antihypertensive compound, captopril, i.e., D-3-mercapto-2-methylpropanoyl-L-proline.

The foregoing prior art compounds are not dipeptide derivatives as are the compounds of the present invention. Furthermore, these prior art compounds contain an essential sulfhydryl substituent or derivative thereof whereas those of the present invention do not. In addition, the dipeptide compounds of the present invention are unusual dipeptides whose N-terminus bears a carboxymethyl group which is preferably further substituted on the methyl group. In addition, the carboxyl group(s) may also be converted to ester, amide and salt derivatives. In effect, the compounds of the present invention are hybrids formed by fusing α-amino acids onto dipeptides by means of a nitrogen shared by these two part-structures. This structural arrangement is rare in the field of synthetic and natural peptides and is not suggested or disclosed by the mercaptoacyl type functions of the two prior art patents identified above.

U.S. Patent 4,052,511 discloses N-carboxyalkanoyl-amino acids which are useful as angiotension converting enzyme inhibitors. Since the compounds of the prsent invention are dipeptide derivatives, in a formal sense they may be considered to be related to some of the compounds disclosed in U.S. Patent 4,052,511. However, when a particular one of the methylene groups is replaced by an amino function as in the present invention, compounds of surprisingly high potency are obtained. For example, the preferred compounds of the present invention can be administered in dosages as low as about 2.5 mg per patient per day as opposed to the lowest dosage level of 1 mg per kg per day for preferred compounds disclosed in the 4,052,511 patent which is about equivalent to 60 mg per patient per day based on an average patient weight of about 150 pounds.

DETAILED DESCRIPTION OF THE INVENTION AND PREFERRED EMBODIMENTS

In its broadest aspect, the present invention relates to novel N-carboxymethyl(amidino)lysyl-proline compounds of the formula:

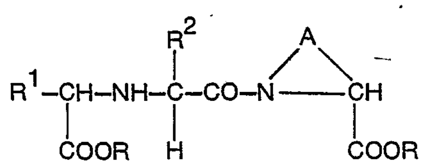

(I)

and

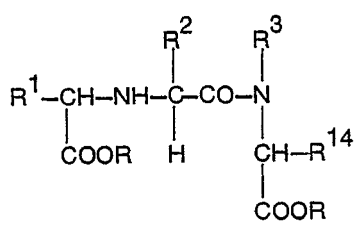

(Ia)

wherein:

R is hydrogen; loweralkyl; aralkyl; or aryl;

$R^1$ is hydrogen; branched or straight chain $C_{1-12}$ alkyl and alkenyl; $C_3$—$C_9$ cycloalkyl and benzofused alkyl; substituted loweralkyl where the substituents are halo, hydroxy loweralkoxy, aryloxy, amino, mono- or diloweralkylamino, acylamino, arylamino, guanidino, mercapto, loweralkylthio, arylthio, carboxy, carboxamido, or loweralkoxycarbonyl; aryl; substituted aryl where the substituents are loweralkyl, loweralkoxy, or halo; aryloweralkyl; arloweralkenyl; heteroarloweralkyl; heteroaryloweralkenyl; substituted arloweralkyl, substituted arloweralkenyl, substituted heteroaryloweralkyl, or substituted heteroarloweralkenyl where the aryl and heteroaryl substituents are halo, dihalo, loweralkyl, hydroxy, loweralkoxy, amino, aminoloweralkyl, acylamino, mono- or diloweralkylamino, carboxyl, haloloweralkyl,

nitro, cyano, or sulfonamido, and where the loweralkyl portion of arloweralkyl may be substituted by amino, acylamino, or hydroxyl;

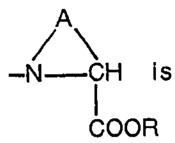 is 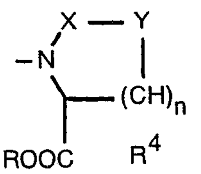 , 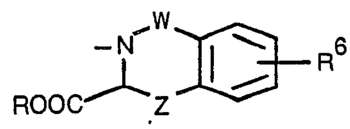

or

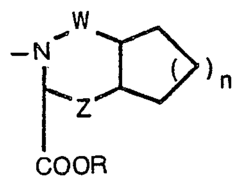

where

X and Y taken together are

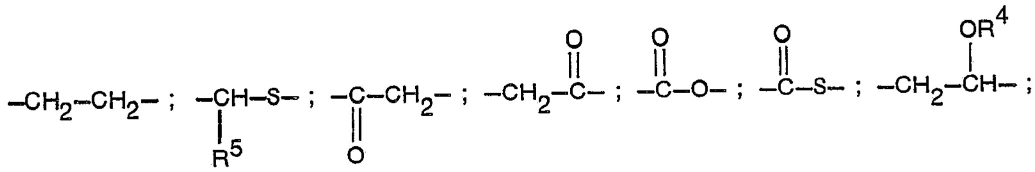

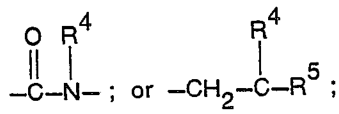

$R^4$ is hydrogen; loweralkyl; aryl; substituted aryl;

$R^5$ is hydrogen; loweralkyl; aryl; or substituted aryl;

n is 1 to 3;

W is absent; $—CH_2—$; or

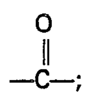

Z is $-(CH_2)_m-$, where m is 0 to 2, provided that m may not be 0 and W may not be absent at the same time; and

$R^6$ is hydrogen; loweralkyl; halo; or $OR^4$;

$R^2$ is $-(CH_2)_r-B-(CH_2)_s-NR^7R^{15}$

where r and s are independently 0 to 3;

B is absent; —O—; —S—; or $—NR^8—$;

where

$R^8$ is hydrogen; loweralkyl; alkanoyl; or aroyl; and

$R^7$ is

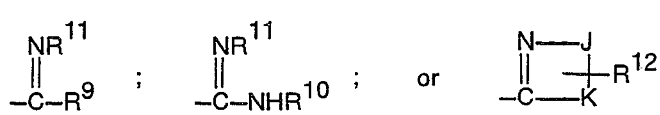

where

$R^9$ is loweralkyl; aralkyl; aryl; heteroaryl; or heteroarloweralkyl; and these groups substituted by hydroxy, lower alkoxy or halo; carboxyl; carboxamido; nitromethenyl;

$R^{10}$ is hydrogen; loweralkyl; aryl; or amidino;

$R^{11}$ is hydrogen; loweralkyl; cyano; amidino; aryl; aroyl; loweralkanoyl;

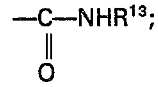

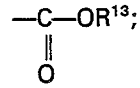

—$NO_2$; —$SO_2NH_2$; or $SO_2R^{13}$;

$R^{12}$ is hydrogen; loweralkyl; halo; aralkyl; amino; cyano; mono- or diloweralkylamino; or $OR^4$;

$R^{13}$ is hydrogen; loweralkyl; or aryl;

$R^{15}$ is hydrogen; lower alkyl; aralkyl; or aryl;

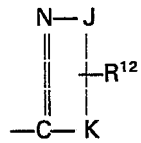

constitute a basic heterocycle of 5 or 6 atoms or benzofused analogs thereof and optionally containing 1—3 N atoms, an oxygen, a sulfur, an S=O, or an $SO_2$ group optionally substituted by amino, lower alkyl amino, diloweralkyl amino, lower alkoxy, or aralkyl groups;

$R^3$ is $C_{3-8}$ cycloalkyl and benzofused $C_{3-8}$ cycloalkyl; aryl; substituted aryl; heteroaryl; substituted heteroaryl;

$R^{14}$ is hydrogen or loweralkyl;

and a pharmaceutically acceptable salt thereof.

The loweralkyl and loweralkenyl substituents recited above represent, except where otherwise indicated, straight and branched chain hydrocarbon radicals of from one to six carbon atoms, such as methyl, ethyl, *n*-propyl, *i*-propyl, *n*-butyl, *i*-butyl, *t*-butyl, *n*-pentyl, *i*-pentyl, *n*-hexyl; or vinyl, allyl, butenyl, and the like.

The loweralkoxy and aryloxy substituents represent loweralkyl and aryl groups as described below attached through an oxygen bridge.

The arloweralkyl and arloweralkenyl substituent's recited above represent phenyl, naphthyl or biphenyl attached through a straight or branched chain hydrocarbon of from one to six carbon atoms, for example, benzyl.

Halo means chloro, bromo, iodo, or fluoro.

The aryl substituent represents phenyl, naphthyl, or biphenyl.

The heteroaryl substituent recited above represents any 5- or 6-membered aromatic ring containing from one to three heteroatoms selected from the group consisting of nitrogen, oxygen, and sulfur, for example, pyridyl, thienyl, furyl, imidazolyl, and thiazolyl; as well as any bicyclic group in which any of the above heterocyclic rings is fused to a benzene ring, for example, indolyl, quinolinyl, isoquinolinyl, benzimidazolyl, benzothiazolyl, and benzthienyl.

Substituted aryl represents aryl rings substituted with hydroxy, lower alkoxy, or halo.

Substituted heteroaryl represents those rings substituted with hydroxy, amino, lower alkoxy, or halo.

The acylamino substituent represents loweralkanoylamino and aroylamino such as, for example, acetylamino or benzoylamino.

Where 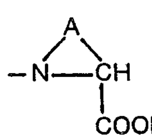 is 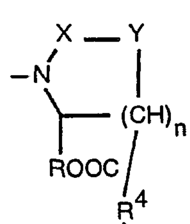 ,

the element: 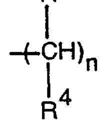

provides for expansion of the ring where n is greater than 1 and $R^4$ is hydrogen, or for loweralkyl substitution of the ring where $R^4$ is loweralkyl, or for any combination of both.

The Formula I compounds can be used in the form of salts derived from inorganic or organic acids. Included among such salts are the following: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfoate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Also, when R is H, salts derived from inorganic or organic bases can be formed. Included among such salts are sodium, potassium, calcium, and ammonium.

Preferred compounds of the present invention are those of Formula I wherein:

R is hydrogen or loweralkyl;

$R^1$ is $C_{1-8}$ alkyl; substituted $C_{1-5}$ alkyl where the substituents are amino, acylamino, arylthio, aryloxy, or arylamino; aralkyl or heteroaralkyl where the alkyl portion has 1 to 4 carbon atoms, for example phenethyl or imidazolylethyl; or substituted aralkyl or heteroaralkyl where the alkyl portion has 1 to 4 carbon atoms and the substituents are halo, dihalo, loweralkyl, hydroxy, loweralkoxy, amino, or aminoloweralkyl, and where the alkyl portion of aralkyl may be substituted by amino, acylamino, or hydroxyl; for

$R^2$, B is absent, r is 2 and s is 1—3 and $R^7$ is

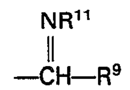

where $R^9$ is loweralkyl; aryl;

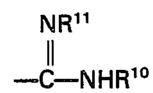

where $R^{10}$ is hydrogen, loweralky, or aryl; and $R^{11}$ is hydrogen, amidino, cyano, nitro, or

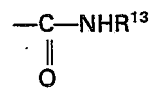

where $R^{13}$ is H, lower alkyl, or aryl;

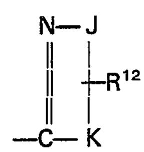

constitute a basic heterocycle of 5 or 6 atoms or benzofused analogs thereof optionally containing 1—3 N atoms, an oxygen, a sulfur, an S=O or an $SO_2$ group.

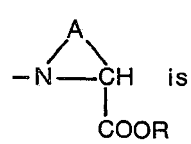 is 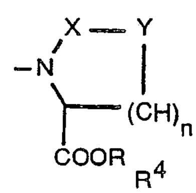

where X and Y taken together are $—CH_2—CH_2—$; $R^4$ is hydrogen; n is 1; and R is as previously defined,

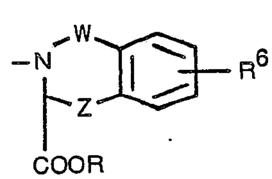

where W is absent or $-CH_2-$; Z is $CH_2$; $-R^6$ is hydrogen; and R is as defined above;

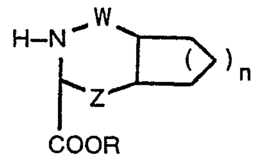

where W is absent or $-CH_2-$; Z is $-CH_2-$; n is 2; and R is as defined above;
$R^3$ is $C_{5-8}$ cycloalkyl and benzofuzed $C_{5-8}$ cycloalkyl;
$R^{14}$ is hydrogen or loweralkyl;
$R^{15}$ is hydrogen or lower alkyl.

Especially preferred compounds of the present invention are the following:
$N^2$-[1-(S)-carboxy-3-phenylpropyl]-$N^6$-(4,5-dihydro-2-thiazolyl)-L-lysyl-L-proline;
$N^2$-[1-(S)-carboxy-3-phenylpropyl]-$N^6$-(1-iminophenyl)-L-lysyl-L-proline;
$N^2$-[1-(S)-carboxy-3-phenylpropyl]-ω-nitro-L-arginyl-L-proline;
$N^2$-[1-(S)-ethoxycarbonyl-3-phenylpropyl]-$N^6$-[(cyanoamino)iminomethyl]-L-lysyl-L-proline;
$N^2$-[1-(S)-carboxy-3-phenylpropyl]-$N^6$-[[(aminoiminomethyl)amino]iminomethyl]-L-lysyl-L-proline;
$N^2$-[1-(S)-ethoxycarbonyl-3-phenylpropyl]-$N^6$-(2-pyrimidinyl)-L-lysyl-L-proline;
$N^2$-[1-(S)-ethoxycarbonyl-3-phenylpropyl]-$N^5$-(2-benzothiazolyl)-L-ornithyl-L-proline;
$N^2$-[1-(S)-carboxy-3-phenylpropyl]-ω-carbamoyl-L-arginyl-L-proline.

The compounds of this invention inhibit angiotensin converting enzyme and thus block conversion of the decapeptide angiotensin I to angiotensin II. Angiotensin II is a potent pressor substance. Thus blood-pressure lowering can result from inhibition of its biosynthesis especially in animals and humans whose hypertension is angiotensin II related. Furthermore, converting enzyme degrades the vasodilator peptide, bradykinin. Therefore, inhibitors of angiotensin converting enzyme may lower blood-pressure also by potentiation of bradykinin. Although the relative importance of these and other possible mechanisms remains to be established, inhibitors of angiotensin converting enzyme are effective antihypertensive agents in a variety of animal models and are useful clinically, for example, in many human patients with renovascular, malignant and essential hypertension. See, for example, D. W. Cushman et al., *Biochemistry 16*, 5484 (1977).

The evaluation of converting enzyme inhibitors is guided by *in vitro* enzyme inhibition assays. For example, a useful method is that of Y. Piquilloud, A. Reinharz and M. Roth, *Biochem. Biophys. Acta, 206,* 136 (1970) in which the hydrolysis of carbobenzyloxyphenylalanylhistidinylleucine is measured. *In vivo* evaluations may be made, for example, in normotensive rats challenged with angiotensin I by the technique of J. R. Weeks and J. A. Jones, *Proc. Soc. Exp. Biol. Med., 104,* 646 (1960) or in a high renin rat model such as that of S. Koletsky et al., *Proc. Soc. Exp. Biol. Med., 125,* 96 (1967).

Thus, the compounds of the invention are useful in treating hypertension. They are also of value in the management of acute and chronic congestive heart failure, in the treatment of secondary hyperaldosteronism, primary and secondary pulmonary hypertension, renal failure and renal vascular hypertension, and in the management of vascular disorders such as migraine. The application of the compounds of this invention for these and similar disorders will be apparent to those skilled in the art.

Thus, in accordance with the present invention there is provided a pharmaceutical composition comprising a pharmaceutically acceptable carrier and a therapeutically effective amount of a compound of Formulae I or Ia.

There is also provided, in accordance with the present invention, a method of treating hypertension comprising administering to a patient in need of such treatment a therapeutically effective amount of a compound of Formulae I or Ia.

For the purpose of treating hypertension, and those clinical conditions noted above, the compounds of the present invention may be administered orally, topically, parenterally, by inhalation spray or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, dogs, cats, etc., the compounds of the invention are effective in the treatment of humans.

The pharmaceutical compositions containing the active ingredient may be in a form suitable for oral use, for example, as tablets, troches, lozenges, aqueous or oily suspensions, dispersible powders or granules, emulsions, hard or soft capsules, syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide a pharmaceutically elegant and palatable preparation. Tablets contain the active ingredient in admixture with non-toxic pharmaceutically acceptable excipients which are suitable for manufacture of tablets. These excipients

may be, for example, inert diluents, such as calcium carbonate, sodium carbonate, lactose, calcium phosphate or sodium phosphate; granulating and disintegrating agents, for example maize starch, or alginic acid; binding agents, for example, starch, gelatine or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate alone or with a wax may be employed.

Formulations for oral use may also be presented as hard gelatine capsules wherein the active ingredient is mixed with an inert solid diluent, for example calcium carbonate, calcium phosphate or kaolin, or as soft gelatine capsules where the active ingredient is mixed with water or an oil medium, for example arachis oil, peanut oil, liquid paraffin or olive oil.

Aqueous suspensions contain the active materials in admixture with excipients suitable for the manufacture of aqueous suspensions. Such excipients are suspending agents, for example sodium carboxymethyl cellulose, methylcellulose, hydroxypropylmethylcellulose, sodium alginate, polyvinylpyrrolidone, gum tragacanth and gum acacia; dispersing or wetting agents may be a natural-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyoxyethylene sorbitan mono-oleate. The said aqueous suspensions may also contain one or more preservatives, for example, ethyl or n-propyl p-hydroxy benzoate, one or more coloring agents, one or more flavoring agents and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as liquid paraffin. The oil suspensions may contain a thickening agent, for example beeswax, hard paraffin or cetyl alcohol. Sweetening agents, such as those set forth above, and flavoring agents may be added to provide a palatable oral preparation. These compositions may be preserved by the addition of an antioxidant such as ascorbic acid.

Dispersible powders and granules suitable for preparation of an aqueous suspension by the addition of water provide the active ingredient in admixture with a dispersing or wetting agent, suspensing agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of oil-in-water emulsions. The oily phase may be a vegetable oil, for exmaple olive oil or arachis oils, or a mineral oil, for example liquid paraffin or mixtures of these. Suitable emulsifying agents may be naturally-occurring gums, for example gum acacia or gum tragacanth, naturally-occurring phosphatides, for example soya bean lecithin, and esters or partial esters derived from fatty acids and hexitol anhydrides, for example sorbitan mono-oleate, and condensation products of the said partial esters with ethylene oxide, for example polyoxyethylene sorbitan mono-oleate. The emulsions may also contain sweetening and flavoring agents.

Syrups and elixirs may be formulated with sweetening agents, for example glycerol, sorbitol or sucrose. Such formulations may also contain a demulcent, a preservative and flavoring and coloring agents. The pharmaceutical compositions may be in the form of a sterile injectable preparation, for example as a sterile injectable aqueous or oleagenous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectibles.

The compounds of this invention may also be administered in the form of suppositories for rectal administration of the drug. These compositions can be prepared by mixing the drug with a suitable nonirritating excipient which is solid at ordinary temperatures by liquid at the rectal temperature and will therefore melt in the rectum to release the drug. Such materials are cocoa butter and polyethylene glycols.

Dosage levels of the order of 5 to 500 mg per patient per day, in single or multiple doses, are useful in the treatment of the above indicated conditions. Preferably, the dosage range will be from 2.5 to 100 mg per day.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general

health, sex, diet, time of administration, route of administration, rate of excretion, drug combination and the severity of the particular disease undergoing therapy.

The compounds of this invention can also be administered in combination with other antihypertensives and/or diuretics. For example, the compounds of this invention can be given in combination with such compounds as amiloride, atenolol, bendroflumethiazide, chlorothalidone, chlorothiazide, clonidine, cryptenamine acetates and cryptenamine tannates, deserpidine, diazoxide, guanethidene sulfate, hydralazine hydrochloride, hydrochlorothiazaide, hydroflumethiazide, indacrinone, metolazone, metoprolol tartate, methylclothiazide, methyldopa, methyldopate hydrochloride, minoxidil, pargyline hydrochloride, polythiazide, prazosin, propranolol, *rauwolfia serpentina,* rescinnamine, reserpine, sodium nitroprusside, spironolactone, timolol, trichlormethiazide, trimethophan camsylate, benzthiazide, quinethazone, ticrynafen, triamterene, acetazolamide, aminophylline, cyclothiazide, ethacrynic acid, furosemide, merethoxylline procaine, sodium ethacrynate, and the like, as well as admixtures and combinations thereof.

Typically, the individual daily dosages for these combinations can range from about one-fifth of the minimally recommended clinical dosages to the maximum recommended levels for the entities when they are given singly.

To illustrate these combinations, one of the antihypertensives of this invention effective in the 2.5 to 100 mg per day range can be effectively combined at levels at the 0.5 to 100 mg per day range with the following compounds at the indicated per day dose range: hydrochlorothiazide (10—100 mg); chlorothiazide (125—2000 mg); manipulated indacrinone enantiomer ratio (25—150 mg); ethacrynic acid (15—2000 mg); amiloride (5—20 mg); furosemide (5—80 mg); propranolol (20—480 mg); timolol (5—60 mg); and methyldopa (65—2000 mg); and the pivaloyloxyethyl ester of methyldopa (30—1000 mg). In addition, triple drug combinations of hydrochlorothiazide (10—100 mg) plus amiloride (5—20 mg) plus converting enzyme inhibitor of this invention (0.5—100 mg); hydrochlorothiazide (10—100 mg) plus timolol (5—60 mg) plus the converting enzyme inhibitor of this invention (0.5—100 mg); or manipulated indacrinone enantiomer ratio (25—150 mg) plus amiloride (5—20 mg) plus converting enzyme inhibitor of this invention (0.5—100 mg) are effective combinations to control blood pressure in hypertensive patients. Naturally, these dose ranges can be adjusted on a unit basis as necessary to permit divided daily dosage and, as noted above, the dose will vary depending on the nature and severity of the disease, weight of patient, special diets and other factors.

Typically, these combinations can be formulated into pharmaceutical compositions as discussed below.

About 0.5 to 100 mg of a compound or mixture of compounds of Formula I or a physiologically acceptable salt is compounded with a physiologically acceptable vehicle, carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

The compounds of Formula I can be prepared by one or more of the methods described further below.

As will be evident to those skilled in the art and as demonstrated in the Examples which follow, reactive groups not involved in the reactions, such as amino, carboxy, mercapto, etc., may be protected by methods standard in peptide chemistry prior to the coupling reactions and subsequently deprotected to obtain the desired products.

A

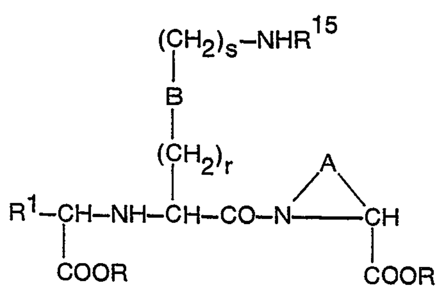

+ REAGENTS

(II)

or

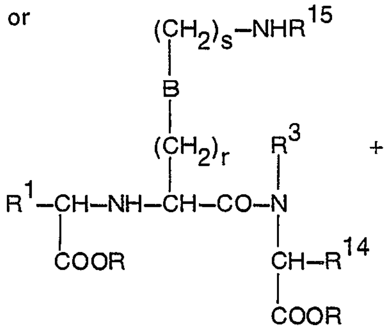

+ REAGENTS

(IIa)

Products of Formulae I and Ia.

REAGENTS :

(1) To afford $R^7$ as 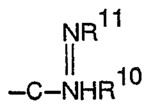 :

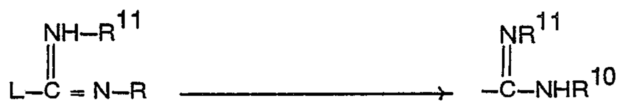

(where L is a leaving group such as methoxy, methylthio, fluoro chloro, and the like) for example:

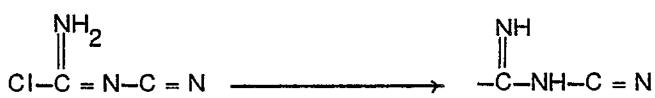

(2) To afford $R^7$ as 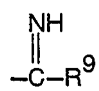 :

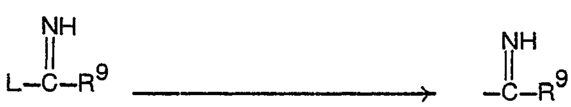

(3) To afford $R^7$ as 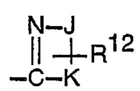 :

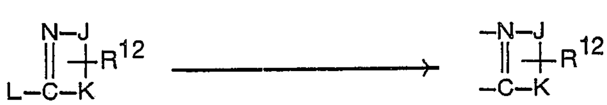

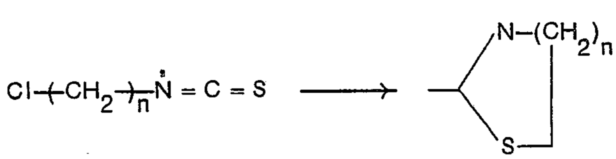

(n is 1 or 2)

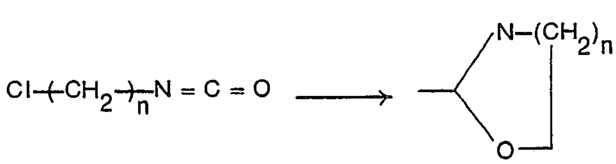

(n is 1 or 2)

In the reactions illustrated above, a starting material of Formula II or IIa is condensed with derivatization reagents to form the desired product. These reagents are familiar to those skilled in the art and are illustrated above as well as in the Examples hereinafter.

The starting materials, which may be prepared in accordance with the procedures described in published European Pat. Appln. No. 0 012 401, are dissolved in a suitable solvent and slightly more than one equivalent of the desired reagent is slowly added, after which the reaction mixture is gently refluxed for several hours. Preferred starting materials are:

$N^2$-[1-(S)-carboxy-3-phenylpropyl]-L-lysyl-L-proline and $N^2$-[1-(S)-carboxy-3-phenylpropyl]-L-ornithyl-L-proline which are prepared in accordance with Examples 57 and 116 of European Pat. Appln. No. 0 012 401.

For compounds of Formula I where $R^7$ is

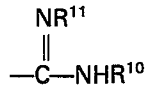 or

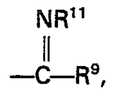,

their preparation may be carried out in accordance with the reaction scheme illustrated below:

B

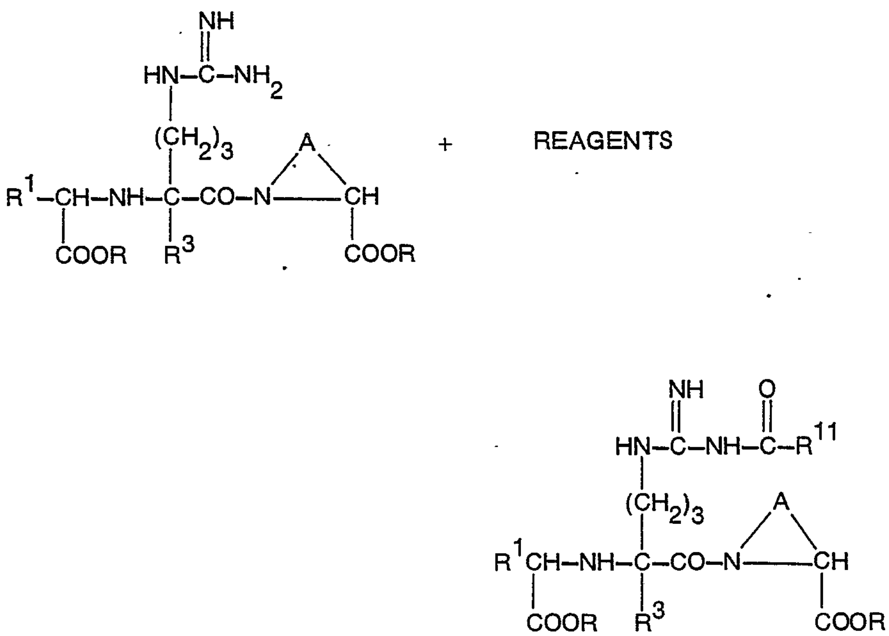


The reagents that can be used in foregoing, illustrative reaction include, for example, benzoylchloride, cyanogen bromide, methyl isocyanate, and benzene sulfonyl chloride. The derivatization reactions are typically conducted in an inert solvent such as DMF in the presence of an aprotic base such as triethylamine. Other reaction conditions are illustrated in the Examples provided hereinbelow.

A preferred starting material is $N^2$-[1-(S)-carboxy-3-phenylpropyl]-L-arginyl-L-proline which can be prepared in accordance with Example 121 of European Pat. Appln. 0 012 401. It is also possible to prepare some of the compounds of this invention by utilizing the derivatized amino acid throughout the entire synthesis sequence. For example, α-carbobenzoyloxy-ω-nitroarginine can be coupled to an amino acid ester and subsequently reductively alkylated with a 2-oxoalkanoic acid to yield, after removal of protecting groups, compounds of Formulae I and Ia in which $R^{11}$ is nitro.

The following examples further illustrate the synthetic routes and reagents which can be used to synthesize compounds of Formulae I and Ia and are intended to be exemplary, but not limitative, of the present invention.

Example 1

$N^2$-[1-(S)-Carboxy-3-phenylpropyl]-$N^6$-(4,5-dihydro-2-thiazolyl)-L-lysyl-L-proline

There was dissolved in 10 ml of methanol and 2 ml of water $N^2$-[1-(S)-carboxy-3-phenylpropyl]-L-lysyl-L-proline (406 mg, 1 mmol). To this solution was added 1 ml of 2-methylthio-2-thiazoline and the mixture was refluxed gently for 20 hours. After cooling, the mixture was diluted with 10 ml of ethylacetate, and then concentrated in vacuo at 50°C to give an oily product. This product was triturated with ethylacetate, and the resulting pale yellow solid was filtered and dried *in vacuo* overnight at 50°C to give 390 mg.

Elemental analysis for $C_{24}H_{34}N_4SO_5.H_2O$

Calc'd: C, 56.68%; H, 7.13%; N, 11.02%

Found: C, 56.76%; H, 7.00%; N, 11.07%

Example 2

$N^2$-[1-(S)-Carboxy-3-phenylpropyl]-$N^6$-(4,5-dihydro-2-thiazolyl)-L-lysyl-L-proline

To 10 ml of a 1:1 mixture of ethanol and water there was added 406 mg (1 mmol) of $N^2$-[1-(S)-carboxy-3-phenylpropyl]-L-lysyl-L-proline. There was then added 0.28 ml of triethylamine (2 mmol) and 122 mg of 2-chloroethylisothiocyanate. The mixture was stirred and refluxed for 18 hrs. A solid settled after one month at room temperature and was triturated with water and ethanol and cooled in an ice bath. A white powder was obtained (141 mg).

Elemental analysis for $C_{24}H_{34}N_4SO_5.H_2O$

Calc'd: C, 56.68%; H, 7.13%; N, 11.02%; S, 6.30%

Found: C, 55.64%; H, 7.14%; N, 10.97%; S, 6.50%

Example 3

$N^2$-[1-(S)-Carboxy-3-phenylpropyl]-$N^6$-(2-pyrimidinyl)-L-lysyl-L-proline

There was dissolved in 4.0 ml of water $N^2$-[1-(S)-carboxy-3-phenylpropyl]-L-lysyl-L-proline (405.5 mg, 1 mmol). To this solution was then added triethylamine (364 mg, 3.6 mmol), followed by slow dropwise addition of a solution of 2-chlorpyrimidine (515.4 mg, 1.5 mmol) in 6.0 ml of ethanol, over 10 mintues with good stirring at room temperature. The reaction mixture was then stirred and refluxed for 15 hours, and then sampled by thin layer chromatography using the system: ethyl acetate/pyridine/acetic acid/water, 10:5:1:3. The results indicated that the reaction was nearly complete. The reaction mixture was refluxed for 2 additional hours and then allowed to stand, after which it was concentrated under vacuum, flushed with benzene, then further concentrated under vacuum. The crude product, a yellow solid, was triturated with benzene, from which a tan solid was filtered off, which, in turn, was washed with benzene and air-dried to give 760 mg. This product was dissolved in 4 ml of methanol and charged to a 2.5 × 240 cm LH-20 column (methanol system) at 20 p.s.i. pressure, taking 14—15 ml fractions every 2 minutes 20 seconds, starting at 40 seconds. Fractions were sampled using the thin layer chromatography system: ethyl acetate/*n*-butanol/acetic acid/water, 1:1:1:1. Fractions 42—45 were combined, dissolved in water, and put through a 6 mg Dowex 50W-X2($H^+$) column washed with 40 ml of water, collecting 8 ml fractions. Fraction 6 having a residue weight of 46.0 mg gave the title compound.

NMR data was consistent with the desired structure. The mass spectrum showed $M^+$=627 m/e (MW + 2 TMS) and 612 m/e, loss of methyl (15).

Example 4

$N^2$-[1-(S)-Carboxy-3-phenylpropyl]-$N^6$-(2-benzothiazolyl)-L-lysyl-L-proline

In 4.0 ml of water there was dissolved $N^2$-[1-(S)-carboxy-3-phenylpropyl]-L-lysyl-L-proline (405 mg, 1 mmol), after which triethylamine (364 mg, 3.6 mmol) was added, followed by slow, dropwise addition of a solution of 2-chlorobenzothiazole (254.5 mg, 1.5 mmol) in 6.0 ml of ethanol. The reaction mixture was stirred at room temperature for 15 minutes, followed by refluxing with stirring for 15 hours. Thin layer chromatography indicated a nearly complete reaction, and refluxing was continued for 2 more hours. The reaction mixture was allowed to cool and then concentrated under vacuum, flushed with benzene 3 times, then further concentrated under vacuum. The crude product (700 mg) was purified on a 2.5 × 240 cm LH-20 chromatographic column using a methanol system. Fractions 42—44 were concentrated (201 mg) and further purified on a chromatographic column in the same manner as described above in Example 3. Fraction 6 with a residue weight of 24.0 mg gave the title compound, and NMR data was consistent with the desired structure. The mass spectrum showed $M^+$ = 565 m/e = 682 ($M^+$ + 2TMS)—117 (—$COOSiMe_3$).

Example 5

$N^2$-[1-(S)-Carboxy-3-phenylpropyl]-$N^6$-(aminoiminomethyl)-L-lysyl-L-proline

The following reactants were combined and heated on a steam bath for 1 hour: $N^2$-[1-(S)-carboxy-3-phenylpropyl]-L-lysyl-L-proline (200 mg, 0.494 mmol); S-methylisothiourea sulfate (99 mg, 0.519 mmol); and 0.624 ml 2N sodium hydroxide (1.25 mmol). After additional heating the pH was adjusted to 7.0, and separation of the product was accomplished on an LH-20 chromatographic column using a $MeCl_2$/MeOH/$H_2O$, 60:40:10 solvent system. Fractions 30—38 (148 mg) were subjected to flash chromatography for

further purification; and fractions 17—24 (10 ml per fraction) gave the title compound (90 mq). NMR data was consistent with the desired structure.

Example 6

$N^2$-[1-(S)-Carboxy-3-phenylpropyl]-$N^6$-(1-iminoethyl)-L-lysyl-L-proline

A. Ethyl acetimidate

In 20 ml of water there was dissolved anhydrous potassium carbonate (4.37 g, 31.6 mmol), after which there was added diethyl ether and then ethyl acetimidate hydrochloride (2.00 g, 16.2 mmol). The reaction mixture was stirred for 10 minutes and the aqueous phase was extracted with 10 ml of diethyl ether. The ether phases were combined and washed with water. The ether phases contained the title compound and were used directly in the next step.

B. $N^2$-[1-(S)-Carboxy-3-phenylpropyl]-$N^6$-(1-iminoethyl)-L-lysyl-L-proline

In 10 ml of ethanol and 2 ml of water there was dissolved $N^2$-[1-(S)-carboxy-3-phenylpropyl]-L-lysyl-L-proline (400 mg, 0.988 mmol). The ether solution from Step A above was added. Additional ethanol was then added to the reaction mixture and it was stirred overnight. The ethanol and diethyl ether were then removed under vacuum after which 10 ml of water and 20 ml of diethyl ether were added. The aqueous phase was freeze dried (649 mg) and then product separation and purification was carried out using an LH-20 chromatographic column in the manner described in Example 3 above, collecting 35 ml fractions, followed by a Dowex chromatographic column purification of fractions 37—39, followed in turn by a further LH-20 chromatographic separation, from which fractions 29—30 gave the title compound (189 mg). NMR data was consistent with the desired structure.

Elemental analysis for $C_{23}H_{34}N_4O_5$:
Calc'd: C, 61.86%; H, 7.67%; N, 12.55%
Found: C, 62.54%; H, 7.85%; N, 12.50

Example 7

$N^2$-[1-(S)-Carboxy-3-phenylpropyl]-$N^6$-(methylaminocyanoiminomethyl)-L-lysyl-L-proline

A. $N^2$-[1-(S)-Carboxy-3-phenylpropyl]-$N^6$-(methylthiocyanoiminomethyl)-L-lysyl-L-proline

There were added together 2 ml of water, 20 ml of ethanol, $N^2$-[1-(S)-carboxy-3-phenylpropyl]-L-lysyl-L-proline (420 mg, 1.04 mmol), and SS'-dimethyl-N-cyanodithioimidocarbonate (300 mg, 2.05 mmol). The resulting clear solution was refluxed for 24 hours at 88°C, then stirred at room temperature for 1.5 days. The reaction mixture was then concentrated under vacuum to an oil (825 mg), which was subsequently put on an LH-20 chromatographic column in the manner described in Example 3 above, collecting 17 ml fractions. Fractions 43—48 were combined and concentrated for use in the next step.

B. $N^2$-[1-(S)-Carboxy-3-phenylpropyl]-$N^6$-(methylaminocyanoiminomethyl)-L-lysyl-L-proline

To the product of Step A (182 mg) was added 2.5 ml of 40% methylamine in water. The resulting clear solution was heated to 60°C overnight, and was then concentrated under vacuum to dryness (201 mg). The product was added to a 20 cc Dowex chromatographic column and eluted with 100 ml water, 8 × 10 ml 2% pyridine/water, and 6 × 25 ml 4% ammonia water. Fractions 6—7 of the pyridine eluent were combined, concentrated to a small volume, and freeze dried (44.1 mg). This product was then placed on an LH-20 chromatographic column in the manner described in Example 3 above, collecting 35 ml fractions. Fractions 43—50 were combined and concentrated, then dissolved in water and freeze dried (24.5 mg) to give the title compound.

Elemental analysis for $C_{24}H_{34}N_6O_5$:
Calc'd: C, 59.24%; H, 7.04%; N, 17.27%
Found: C, 58.70%; H, 6.81%; N, 17.08%

Example 8

$N^2$-[1-(S)-Carboxy-3-phenylpropyl]-$N^6$-(3-amino-1H-1,2,4-triazolyl)-L-lysyl-L-proline

This compound was prepared in a manner similar to the preparation of $N^2$-[1-(S)-carboxy-3-phenylpropyl]-$N^6$-(methylaminocyanoiminomethyl)-L-lysyl L-proline, except that 90% ethanol (20 ml) was used. From 290 mg of $N^2$-[1-(S)-carboxy-3-phenylpropyl]-$N^6$-(methylthiocyanoiminomethyl)-L-lysyl-L-proline and 0.5 ml of 95% hydrazine, 21.0 mg of $N^2$-[1-(S)-carboxy-3-phenylpropyl]-$N^6$(3-amino-1H-1,2,4-triazolyl)-L-lysyl-L-proline was obtained upon chromatography on Dowex 50W-2X using 3% pyridine in water, a white solid on freeze-drying. The highest mass (FAB) is 469 m/e, which is loss of water (18) from the desired production ion M+ (487).

Example 9

Using known amino acids and known substituted 2-oxoalkanoic acids or their esters and employing the methods taught in European Pat. Appln. No. 0 012 401, it is possible to synthesize starting materials for derivatization such as those listed in Table I below.

Representative derivatization reagents are shown in Table II below. Somewhat over an equivalent of

each are brought into the reaction with an equivalent amount of a starting material shown in Table I to yield additional products of the invention listed in Table III below. The derivatization conditions often involve the presence of excess base and are standard for the use of the various reagents as taught in the literature for the use in derivatizing amines, amidines or guanidines.

TABLE I

Starting Materials for Derivatization

1)

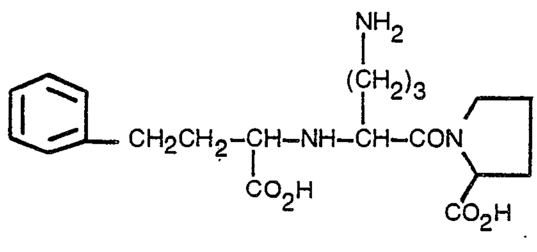

2)

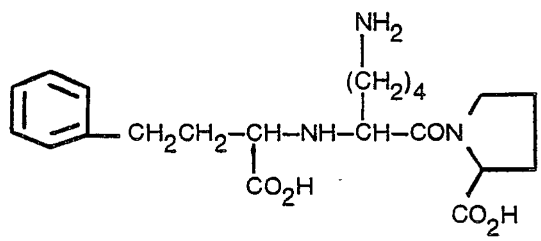

3)

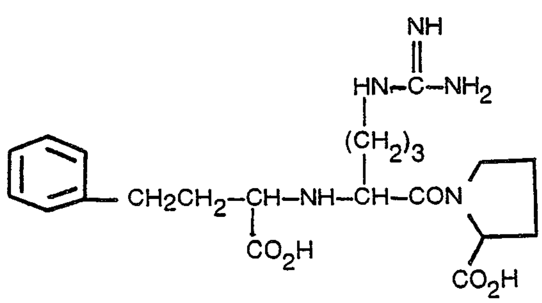

4)

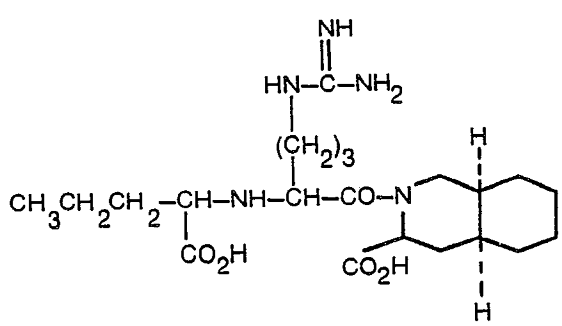

5)

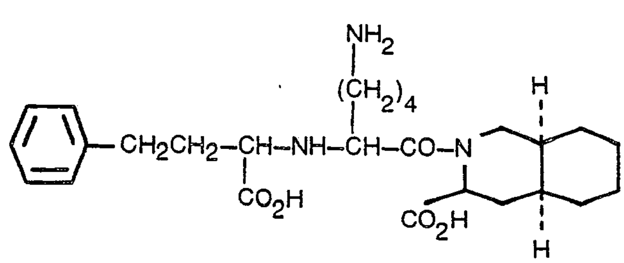

TABLE I (Continued)

Starting Materials for Derivatization

6) 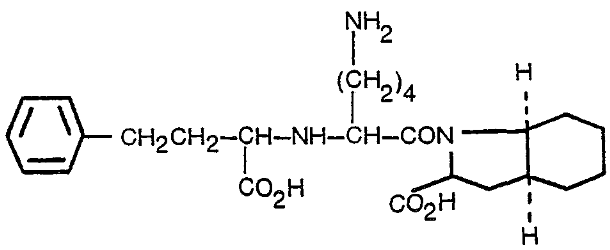

7) 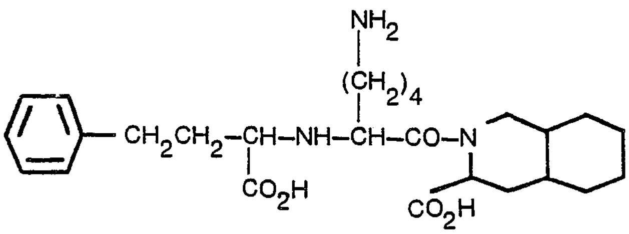

8) 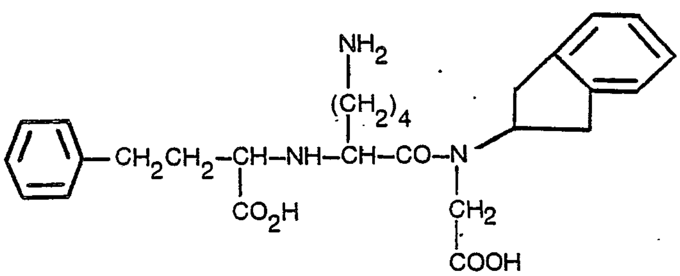

9) 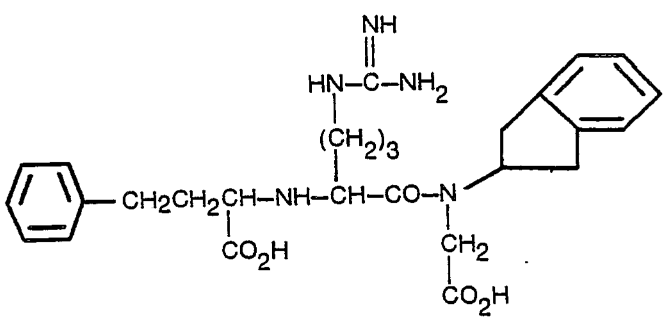

10) 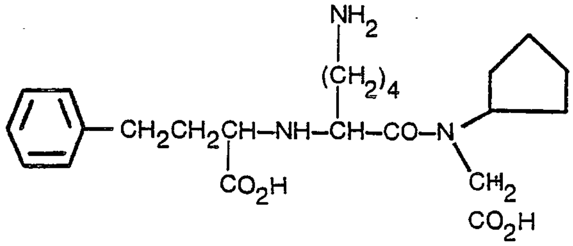

TABLE I (Continued)

Starting Materials for Derivatization

11) 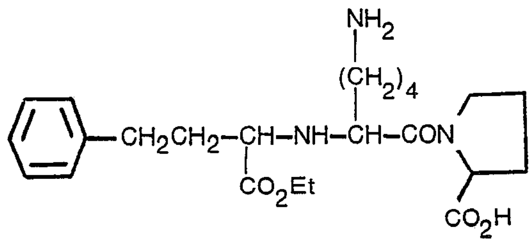

12) 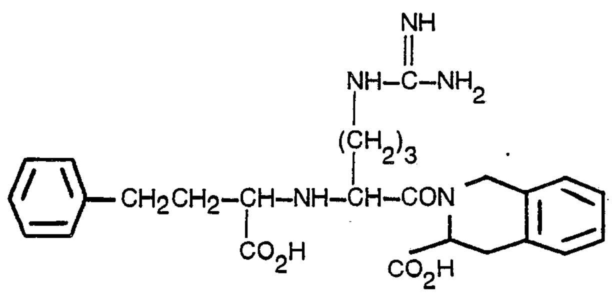

TABLE II

Reagents

I. Especially for Derivatizing Guanidines and Amidines

a) $CH_3COCl$

b) 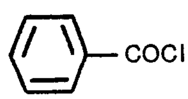

c) $Cl{-}SO_2N = C = O$

d) $ClSO_2NH_2$

e) $CH_3N = C = O$

f) 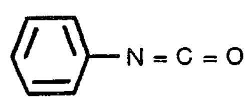

g) $C_2H_5O{-}COCl$

h) $CH_3SO_2Cl$

i) 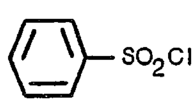

TABLE II (Continued)
Reagents

II. Especially for Derivatizing Guanidines and Amidines

j) 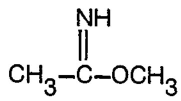

k) 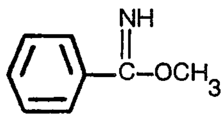

l) $Cl-CH_2CH_2N=C=S$

m) $Cl-CH_2CH_2N=C=O$

n) $(CH_3)_2N^+=CHCl$

o) 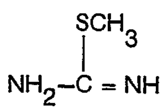

p) 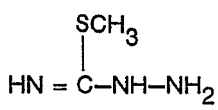

q) $HN-(CN)_2$

r) 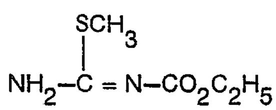

s) 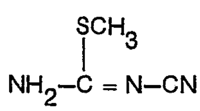

t) 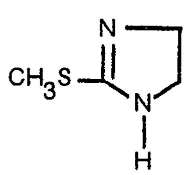

u) 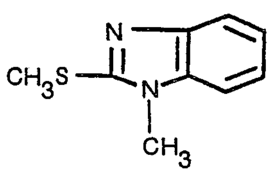

TABLE II (Continued)
Reagents

II. Especially for Derivatizing Guanidines and Amidines

v) $NH_2C \equiv N$

w) 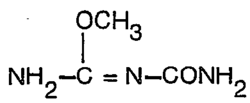

x) 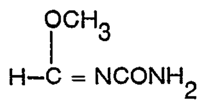

y) 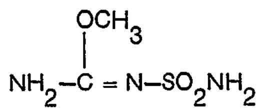

z) 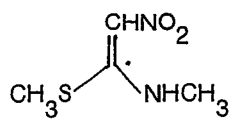

aa) 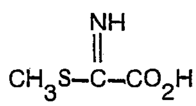

bb) 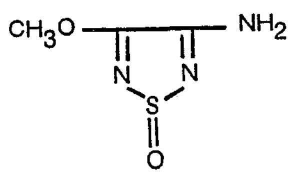

cc) 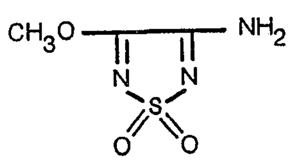

dd) 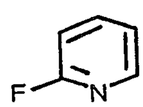

ee) 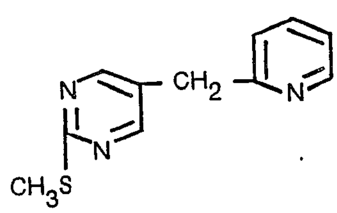

TABLE II (Continued)

Reagents

II. Especially for Derivatizing Guanidines and Amidines

ff) 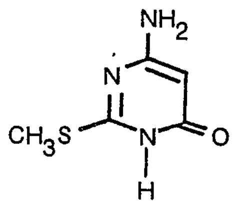

gg) 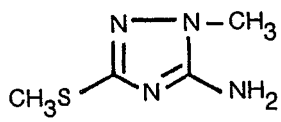

TABLE III

Compounds of Formulae I and Ia

A) 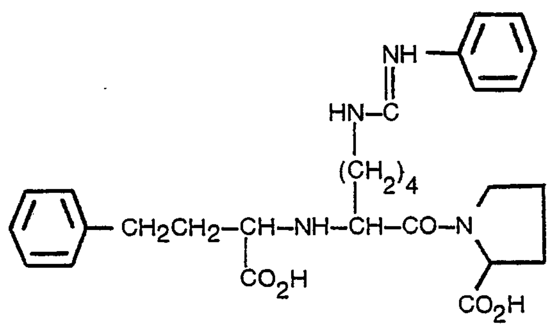

B) 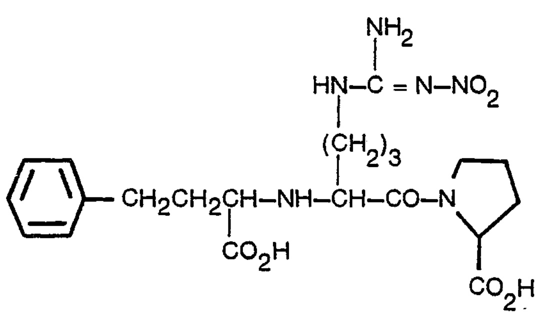

C) 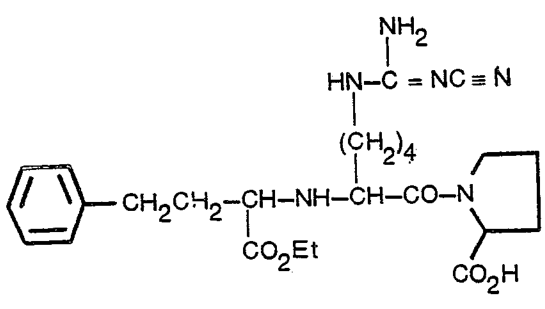

TABLE III (Continued)

Compounds of Formulae I and Ia

D)

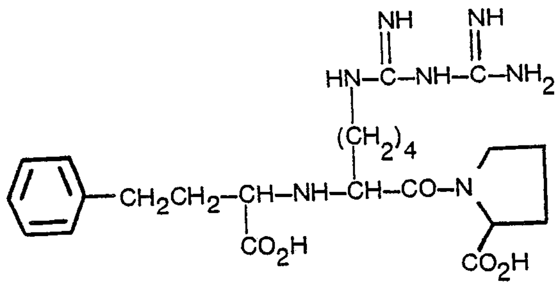

E)

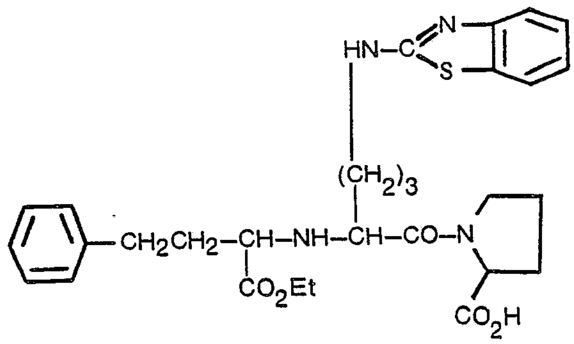

F)

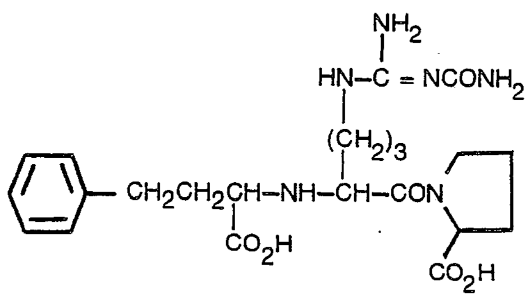

G)

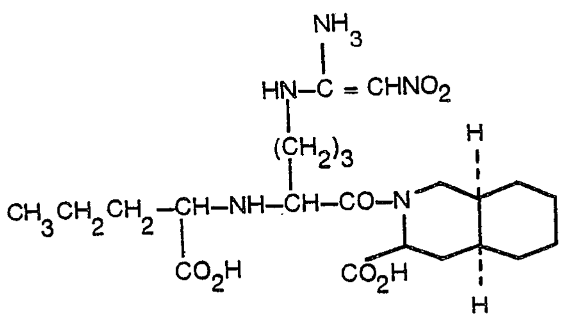

H)

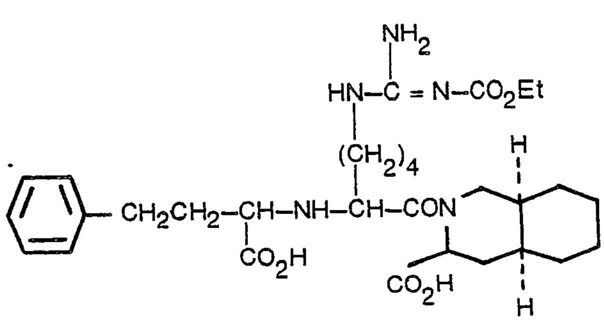

TABLE III (Continued)

Compounds of Formulae I and Ia

I)

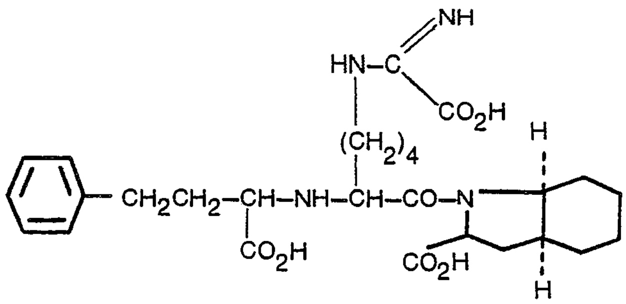

J)

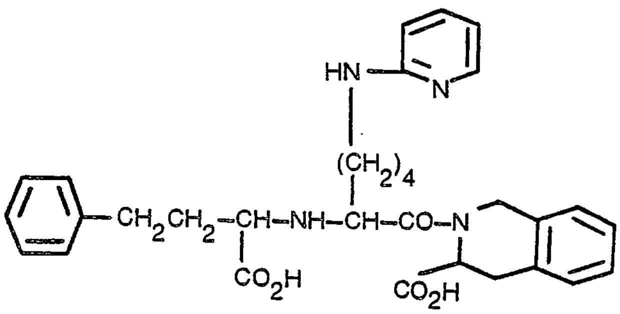

K)

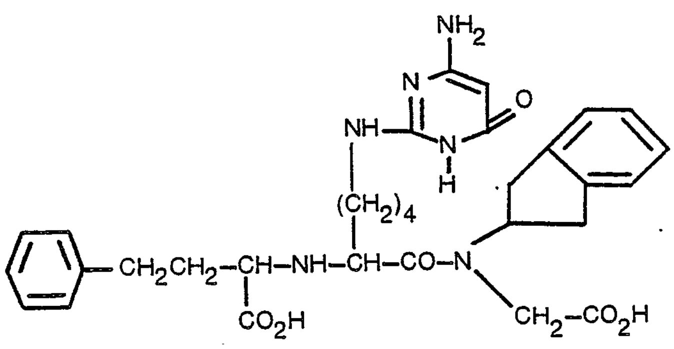

L)

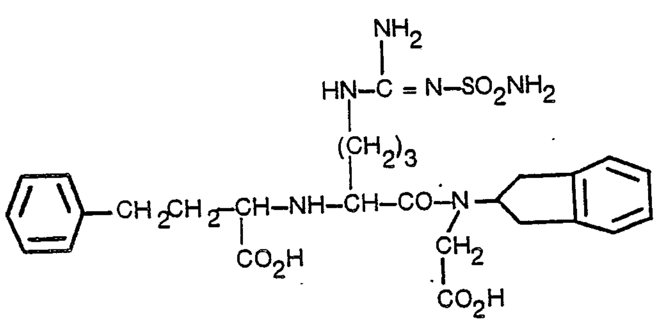

TABLE III (Continued)

Compounds of Formulae I and Ia

M) 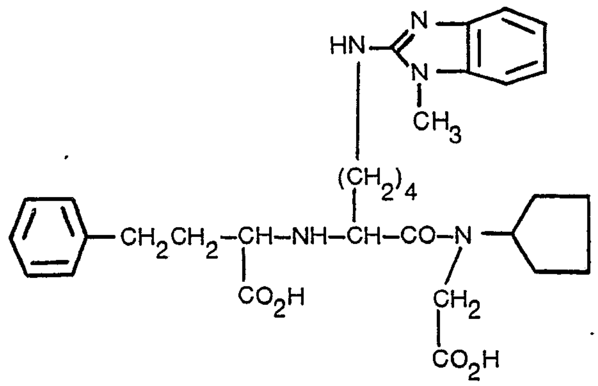

N) 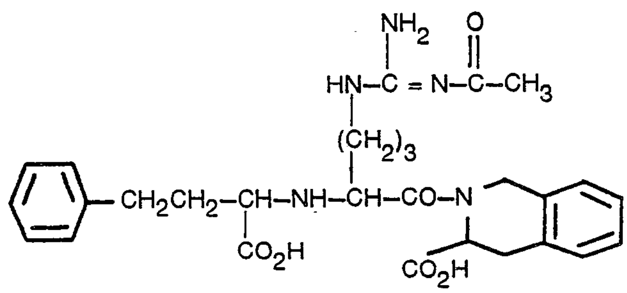

O) 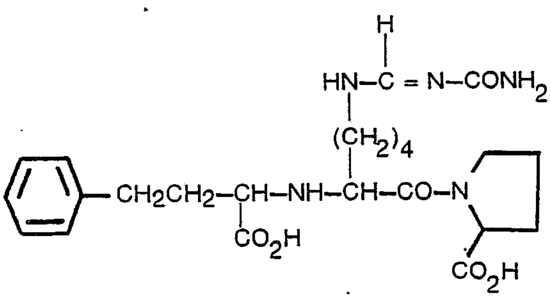

## Claims

1. Compounds of the formulae:

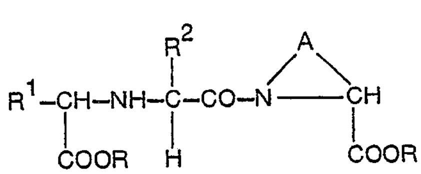

and

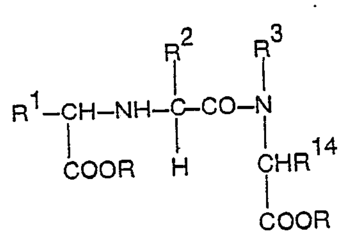

(I) (Ia)

wherein:

R is hydrogen; loweralkyl; aralkyl; or aryl;

$R^1$ is hydrogen; branched or straight chain $C_{1-12}$ alkyl and alkenyl; $C_3$—$C_9$ cycloalkyl and benzofused alkyl; substituted loweralkyl where the substituents are halo, hydroxy loweralkoxy, aryloxy, amino, mono-or diloweralkylamino, acylamino, arylamino, guanidino, mercapto, loweralkylthio, arylthio, carboxy, carboxamido, or loweralkoxycarbonyl; aryl; substituted aryl where the substituents are loweralkyl, loweralkoxy, or halo; aryloweralkyl; arloweralkenyl; heteroarloweralkyl; heteroaryloweralkenyl; substituted arloweralkyl, substituted arloweralkenyl, substituted heteroaryloweralkyl, or substituted heteroarloweralkenyl where the aryl and heteroaryl substituents are halo, dihalo, loweralkyl, hydroxy, loweralkoxy, amino, aminoloweralkyl, acylamino, mono- or diloweralkylamino, carboxyl, haloloweralkyl, nitro, cyano, or sulfonamido, and where the loweralkyl portion of arloweralkyl may be substituted by amino, acylamino, or hydroxyl;

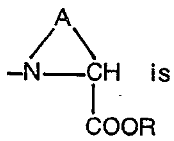 is 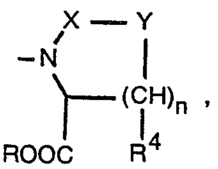 , 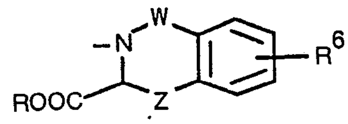

or

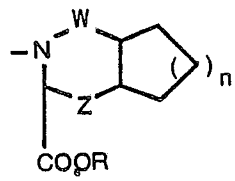

where

X and Y taken together are

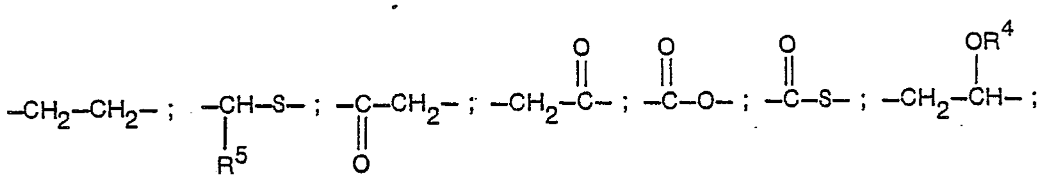

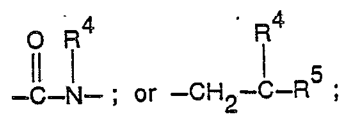

$R^4$ is hydrogen; loweralkyl; aryl; substituted aryl;

$R^5$ is hydrogen; loweralkyl; aryl; or substituted aryl;

n is 1 to 3;

W is absent; $—CH_2—$; or

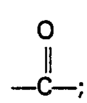

Z is $-(CH_2)_m-$, where m is 0 to 2, provided that m may not be 0 and W may not be absent at the same time; and

$R^6$ is hydrogen; loweralkyl; halo; or $OR^4$;

$R^2$ is $-(CH_2)_r-B-(CH_2)_s-NR^7R^{15}$

where r and s are independently 0 to 3;

B is absent; —O—; —S—; or —$NR^8$—;

where

$R^8$ is hydrogen; loweralkyl; alkanoyl; or aroyl; and

$R^7$ is

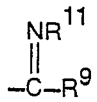 ; 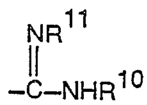 ; or 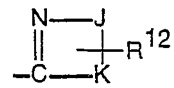

where

$R^9$ is loweralkyl; aralkyl; aryl; heteroaryl; or heteroarloweralkyl; and these groups substituted by hydroxy, lower alkoxy or halo; carboxyl; carboxamido; nitromethenyl;

$R^{10}$ is hydrogen; loweralkyl; aryl; or amidino;

$R^{11}$ is hydrogen; loweralkyl; cyano; amidino; aryl; aroyl; loweralkanoyl;

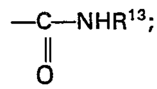

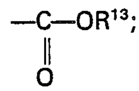

—$NO_2$; —$SO_2NH_2$; or $SO_2R^{13}$;

$R^{12}$ is hydrogen; loweralkyl; halo; aralkyl; amino; cyano; mono- or diloweralkylamino; or $OR^4$;

$R^{13}$ is hydrogen; loweralkyl; or aryl;

$R^{15}$ is hydrogen; lower alkyl; aralkyl; or aryl;

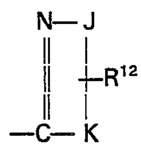

constitute a basic heterocycle of 5 or 6 atoms or benzofused analogs thereof and optionally containing 1—3 N atoms, an oxygen, a sulfur, an S=O, or an $SO_2$ group optionally substituted by amino, lower alkyl amino, diloweralkyl amino, lower alkoxy, or aralkyl groups;

$R^3$ is $C_{3-8}$ cycloalkyl and benzofused $C_{3-8}$ cycloalkyl; perhydrobenzofused $C_{3-8}$ cycloalkyl; aryl; substituted aryl; heteroaryl; substituted heteroaryl;

$R^{14}$ is hydrogen or loweralkyl;

and a pharmaceutically acceptable salt thereof.

2. A compound of Claim 1 which is a member of the group:

$N^2$-[1-(S)-carboxy-3-phenylpropyl]-$N^6$-(4,5-dihydro-2-thiazolyl)-L-lysyl-L-proline;

$N^2$-[1-(S)-carboxy-3-phenylpropyl]-$N^6$-(1-iminophenyl)-L-lysyl-L-proline;

$N^2$-[1-(S)-carboxy-3-phenylpropyl]-ω-nitro-L-arginyl-L-proline;

$N^2$-[1-(S)-ethoxycarbonyl-3-phenylpropyl]-$N^6$-[(cyanoamino)-iminomethyl]-L-lysyl-L-proline;

$N^2$-[1-(S)-carboxy-3-phenylpropyl]-$N^6$-[[(aminoiminomethyl)amino]iminomethyl]-L-lysyl-L-proline;

$N^2$-[1-(S)-ethoxycarbonyl-3-phenylpropyl]-$N^6$-(2-pyrimidinyl)-L-lysyl-L-proline;

$N^2$-[1-(S)-ethoxycarbonyl-3-phenylpropyl]-$N^5$-(2-benzothiazolyl)-L-ornithyl-L-proline; and

$N^2$-[1-(S)-carboxy-3-phenylpropyl]-ω-carbamoyl-L-arginyl-L-proline.

3. A pharmaceutical composition useful in treating hypertension comprising a pharmaceutically acceptable carrier and an antihypertensively effective amount of a compound of the formulae:

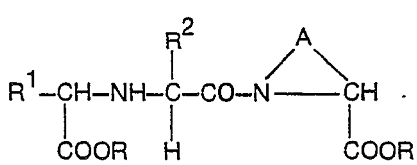

(I)

and

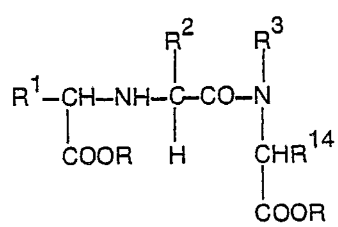

(Ia)

wherein:

R is hydrogen; loweralkyl; aralkyl; or aryl;

$R^1$ is hydrogen; branched or straight chain $C_{1-12}$ alkyl and alkenyl; $C_3$—$C_9$ cycloalkyl and benzofused alkyl; substituted loweralkyl where the substituents are halo, hydroxy loweralkoxy, aryloxy, amino, mono- or diloweralkylamino, acylamino, arylamino, guanidino, mercapto, loweralkylthio, arylthio, carboxy, carboxamido, or loweralkoxycarbonyl; aryl; substituted aryl where the substituents are loweralkyl, loweralkoxy, or halo; aryloweralkyl; arloweralkenyl; heteroarloweralkyl; heteroaryloweralkenyl; substituted arloweralkyl, substituted arloweralkenyl, substituted heteroaryloweralkyl, or substituted heteroarloweralkenyl where the aryl and heteroaryl substituents are halo, dihalo, loweralkyl, hydroxy, loweralkoxy, amino, aminoloweralkyl, acylamino, mono- or diloweralkylamino, carboxyl, haloloweralkyl, nitro, cyano, or sulfonamido, and where the loweralkyl portion of arloweralkyl may be substituted by amino, acylamino, or hydroxyl;

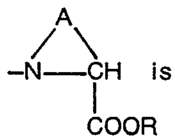 is 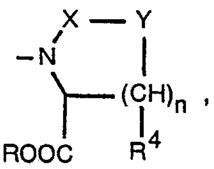 , 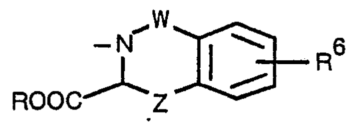

or

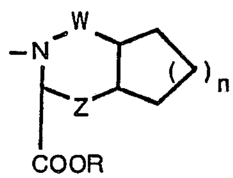

where

X and Y taken together are

$-CH_2-CH_2-$ ; 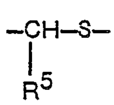 ; 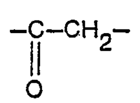 ; 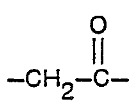 ; 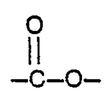 ; 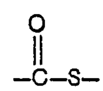 ; 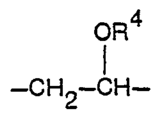 ;

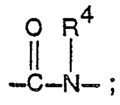 ; or 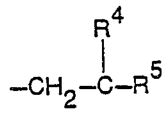 ;

$R^4$ is hydrogen; loweralkyl; aryl; substituted aryl;

$R^5$ is hydrogen; loweralkyl; aryl; or substituted aryl;

n is 1 to 3;

W is absent; —$CH_2$—; or

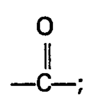

Z is $-(CH_2)_m-$, where m is 0 to 2, provided that m may not be 0 and W may not be absent at the same time; and

$R^6$ is hydrogen; loweralkyl; halo; or $OR^4$;

$R^2$ is $-(CH_2)_r-B-(CH_2)_s-NR^7R^{15}$

where r and s are independently 0 to 3;

B is absent; —O—; —S—; or —$NR^8$—;
where
$R^8$ is hydrogen; loweralkyl; alkanoyl; or aroyl; and
$R^7$ is

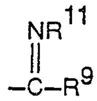 ; 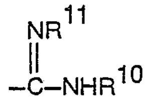 ; or 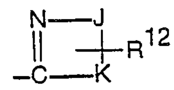

where
$R^9$ is loweralkyl; aralkyl; aryl; heteroaryl; or heteroarloweralkyl; and these groups substituted by hydroxy, lower alkoxy or halo; carboxyl; carboxamido; nitromethenyl;
$R^{10}$ is hydrogen; loweralkyl; aryl; or amidino;
$R^{11}$ is hydrogen; loweralkyl; cyano; amidino; aryl; aroyl; loweralkanoyl;

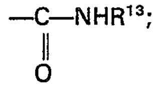

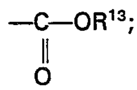

—$NO_2$; —$SO_2NH_2$; or $SO_2R^{13}$;
$R^{12}$ is hydrogen; loweralkyl; halo; aralkyl; amino; cyano; mono- or diloweralkylamino; or $OR^4$;
$R^{13}$ is hydrogen; loweralkyl; or aryl;
$R^{15}$ is hydrogen; lower alkyl; aralkyl; or aryl;

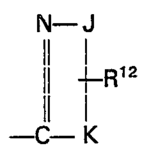

constitute a basic heterocycle of 5 or 6 atoms or benzofused analogs thereof and optionally containing 1—3 N atoms, an oxygen, a sulfur, an S=O, or an $SO_2$ group optionally substituted by amino, lower alkyl amino, diloweralkyl amino, lower alkoxy, or aralkyl groups;
$R^3$ is $C_{3-8}$ cycloalkyl and benzofused $C_{3-8}$ cycloalkyl; perhydrobenzofused $C_{3-8}$ cycloalkyl; aryl; substituted aryl; heteroaryl; substituted heteroaryl;
$R^{14}$ is hydrogen or loweralkyl; and a pharmaceutically acceptable salt thereof.

4. A pharmaceutical composition useful in the treatment of hypertension which comprises a pharmaceutically acceptable carrier; an anti-hypertensively effective amount of a compound of Claim 1; and, an antihypertensive and/or diuretic compound selected from the group consisting of amiloride, atenolol, bendroflumethiazide, chlorothalidone, chlorothiazide, clonidine, cryptenamine acetates and cryptenamine tannates, deserpidine, diazoxide, guanethidene sulfate, hydralazine hydrochloride, hydrochlorothiazaide, hydroflumethiazide, indacrinone, metolazone, metoprolol tartrate, methylclothiazide, methyldopa, methyldopate hydrochloride, minoxidil, pargyline hydrochloride, polythiazode, prazosin, propranolol, *rauwolfia serpentina,* rescinnamine, reserpine, sodium nitroprusside, spironolactone, timolol, trichlormethiazide, trimethophan camsylate, benzthiazide, guinethazone, ticrynafen, triamterene, acetazolamide, aminophylline, cyclothiazide, ethacrynic acid, furosemide, merethoxylline procaine and sodium ethacrynate.

5. A process for preparing Formulae I and Ia compounds of Claim 1 which comprises derivatization with appropriate reagents of substituted N-carboxymethyl dipeptides having the formulae:

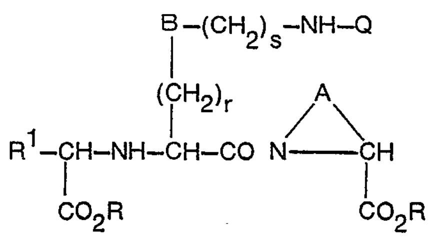

and

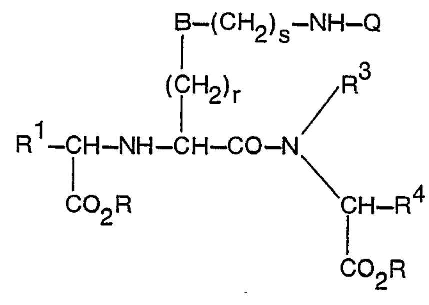

wherein Q is H or

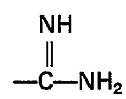

and R, $R^1$, B, r, s, $R^3$ and $R^4$ are as defined in Claim 1 to obtain compounds of Formulae I and Ia and, if desired, removing protecting groups if present by standard methods and, if further desired, preparing salts of said compounds by conventional means.

**Patentansprüche**

1. Verbindungen der Formeln

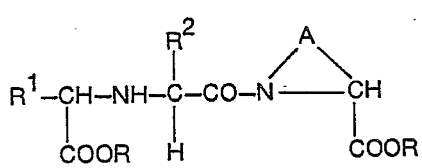

(I)

und

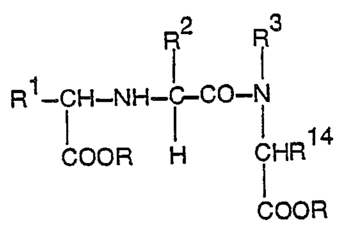

(Ia)

worin:

R ist Wasserstoff; nieder-Alkyl; Aralkyl; oder Aryl;

$R^1$ ist Wasserstoff; verzweigtes oder geradkettiges $C_1$—$_{12}$-Alkyl und -Alkenyl; $C_3$—$C_9$-Cycloalkyl und benzokondensiertes Alkyl; substituiertes nieder-Alkyl, wobei es sich bei den Substituenten um Halogen, Hydroxy, nieder-Alkoxy, Aryloxy, Amino, Mono- oder Di-nieder-alkylamino, Acylamino, Arylamino, Guanidino, Mercapto, nieder-Alkylthio, Arylthio, Carboxy, Carboxamido oder nieder-Alkoxycarbonyl handelt; Aryl; substituiertes Aryl, wobei es sich bei den Substituenten um nieder-Alkyl, nieder-Alkoxy oder Halogen handelt; Ar-nieder-alkyl; Ar-nieder-alkenyl; Heteroar-nieder-alkyl; Heteroar-nieder-alkenyl; substituiertes Ar-nieder-alkyl; substituiertes Ar-nieder-alkenyl, substituiertes Heteroar-nieder-alkyl oder substituiertes Heteroar-nieder-alkenyl, wobei es sich bei den Aryl- und Heteroaryl-substituenten um Halogen, Dihalogen, nieder-Alkyl, Hydroxy, nieder-Alkoxy, Amino, Amino-nieder-alkyl, Acylamino, Mono- oder Di-nieder-alkylamino, Carboxyl, Halogen-nieder-alkyl, Nitro, Cyano oder Sulfonamido handelt und wobei der nieder-Alkylbereich von Ar-nieder-alkyl durch Amino, Acylamino oder Hydroxyl substituiert sein kann:

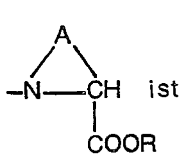 ist 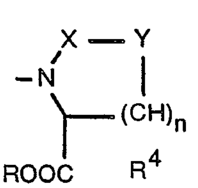, 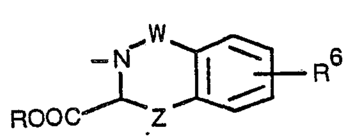 oder 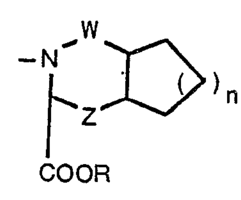

wobei X und Y zusammen

$-CH_2-CH_2-$ ; 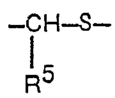 ; 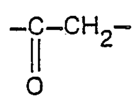 ; 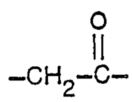 ; 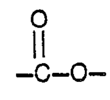 ; 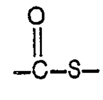 ; 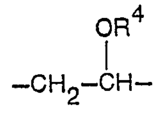 ;

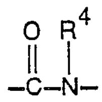 ; oder 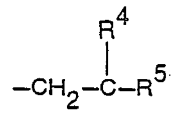 ;

bedeuten:

$R^4$ ist Wasserstoff; nieder-Alkyl; Aryl; substituiertes Aryl;

$R^5$ ist Wasserstoff; nieder-Alkyl; Aryl; oder substituiertes Aryl;

n ist 1 bis 3;

W ist abwesend; $—CH_2—$; oder

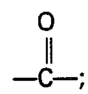;

Z ist $-(CH_2)_m-$, wobei m 0 bis 2 ist,
mit der Maßgabe, daß nicht gleichzeitig m 0 ist und W abwesend ist; und

$R^6$ ist Wasserstoff; nieder-Alkyl; Halogen; oder $OR^4$;

$R^2$ ist $-(CH_2)_r-B-(CH_2)_s-NR^7R^{15}$

wobei

r und s unabhängig voneinander 0 bis 3 sind;

B ist abwesend; —O—; —S—; oder $—NR^8—$;

wobei $R^8$ Wasserstoff, nieder-Alkyl; Alkanoyl; oder Aroyl ist; und

$R^7$ ist

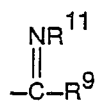 ; 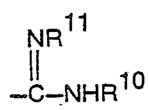 ; . oder 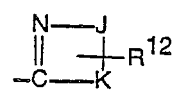

worin

$R^9$ ist nieder-Alkyl; Aralkyl; Aryl, Heteroaryl; oder Heteroar-nieder-alkyl und diese Gruppen substituiert durch Hydroxyl, nieder-Alkoxy oder Halogen; Carboxyl; Carboxamido; Nitromethenyl;

$R^{10}$ ist Wasserstoff; nieder-Alkyl; Aryl; oder Amidino;

$R^{11}$ ist Wasserstoff; nieder-Alkyl; Cyano; Amidino; Aryl; Aroyl; nieder-Alkanoyl;

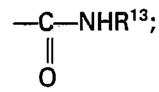;

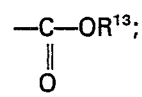;

$—NO_2$; $—SO_2NH_2$; oder $SO_2R^{13}$;

$R^{12}$ ist Wasserstoff; nieder-Alkyl; Halogen; Aralkyl; Amino; Cyano; Mono- oder Di-nieder-alkylamino; oder $OR^4$;

$R^{13}$ ist Wasserstoff; nieder-Alkyl; oder Aryl;

$R^{15}$ ist Wasserstoff; nieder-Alkyl; Aralkyl; oder Aryl;

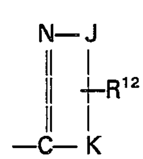

stellt einen basischen Heterocyclus mit 5 oder 6 Atomen oder benzokondensierte Analoge davon, die ggf. 1 bis 3 N-Atome, eine Sauerstoff-, eine Schwefel-, eine S=O— oder eine $SO_2$-Gruppe enthalten und ggf. durch Amino-, nieder-Alkylamino-, Di-nieder-alkylamino-, nieder-Alkoxy- oder Aralkylgruppen substituiert sein können, dar;

$R^3$ ist $C_3$—$C_8$-Cycloalkyl und benzokondensiertes $C_3$—$C_8$-Cycloalkyl; perhydrobenzokondensiertes $C_3$—$C_8$-Cycloalkyl; Aryl; substituiertes Aryl; Heteroaryl; substituiertes Heteroaryl;

$R^{14}$ ist Wasserstoff oder nieder-Alkyl; und pharmazeutisch verträgliche Salze davon.

2. Eine Verbindung nach Anspruch 1, die ein Bestandteil aus folgender Gruppe ist:
$N^2$-[1-(S)-Carboxy-3-phenylpropyl]-$N^6$-(4,5-dihydro-2-thiazolyl)-L-lysyl-L-prolin;
$N^2$-[1-(S)-Carboxy-3-phenylpropyl]-$N^6$-(1-iminophenyl)-L-lysyl-L-prolin;
$N^2$-[1-(S)-Carboxy-3-phenylpropyl]-ω-nitro-L-arginyl-L-prolin;
$N^2$-[1-(S)-Äthoxycarbonyl-3-phenylpropyl]-$N^6$-[(cyanoamino)-iminomethyl]-L-lysyl-L-prolin;
$N^2$-[1-(S)-Carboxy-3-phenylpropyl]-$N^6$-[[(aminoiminomethyl)-amino]-iminomethyl]-L-lysyl-L-prolin;
$N^2$-[1-(S)-Äthoxycarbonyl-3-phenylpropyl]-$N^6$-(2-pyrimidinyl)-L-lysyl-L-prolin;
$N^2$-[1-(S)-Äthoxycarbonyl-3-phenylpropyl]-$N^5$-(2-benzothiazolyl)-L-ornithyl-L-prolin; und
$N^2$-[1-(S)-Carboxy-3-phenylpropyl]-ω-carbamoyl-L-arginyl-L-prolin.

3. Eine pharmazeutische Zusammensetzung zur Behandlung von Hochdruck, enthaltend einen pharmazeutisch verträglichen Trägerstoff und eine antihypertensiv wirkende Menge einer Verbindung der Formeln

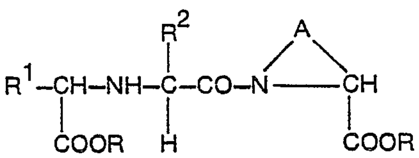

(I)

und

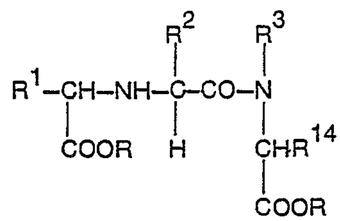

(Ia)

worin:

R ist Wasserstoff; nieder-Alkyl; Aralkyl; oder Aryl;

$R^1$ ist Wasserstoff; verzweigtes oder geradkettiges $C_1$—$_{12}$-Alkyl und -Alkenyl; $C_3$—$C_9$-Cycloalkyl und benzokondensiertes Alkyl; substituiertes nieder-Alkyl, wobei es sich bei den Substituenten um Halogen, Hydroxy, nieder-Alkoxy, Aryloxy, Amino, Mono- oder Di-nieder-alkylamino, Acylamino, Arylamino, Guanidino, Mercapto, nieder-Alkylthio, Arylthio, Carboxy, Carboxamido oder nieder-Alkoxycarbonyl handelt; Aryl; substituiertes Aryl, wobei es sich bei den Substituenten um nieder-Alkyl, nieder-Alkoxy oder Halogen handelt; Ar-nieder-alkyl; Ar-nieder-alkenyl; Heteroar-nieder-alkyl; Heteroar-nieder-alkenyl; substituiertes Ar-nieder-alkyl; substituiertes Ar-nieder-alkenyl, substituiertes Heteroar-nieder-alkyl oder substituiertes Heteroar-nieder-alkenyl, wobei es sich bei den Aryl- und Heteroaryl-substituenten um Halogen, Dihalogen, nieder-Alkyl, Hydroxy, nieder-Alkoxy, Amino, Amino-nieder-alkyl, Acylamino, Mono- oder Di-nieder-alkylamino, Carboxyl, Halogen-nieder-alkyl, Nitro, Cyano oder Sulfonamido handelt und wobei der nieder-Alkylbereich von Ar-nieder-alkyl durch Amino, Acylamino oder Hydroxyl substituiert sein kann:

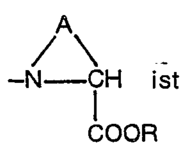

ist

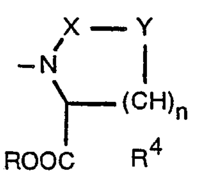

,

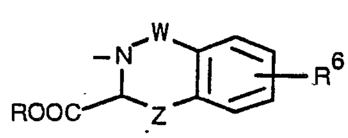

oder

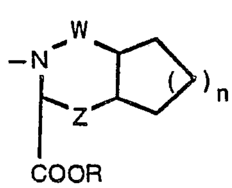

wobei X und Y zusammen

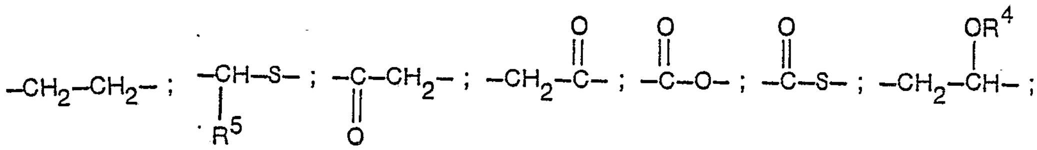

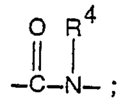 oder 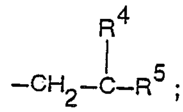

bedeuten:

$R^4$ ist Wasserstoff; nieder-Alkyl; Aryl; substituiertes Aryl;

$R^5$ ist Wasserstoff; nieder-Alkyl; Aryl; oder substituiertes Aryl;

n ist 1 bis 3;

W ist abwesend; $—CH_2—$; oder

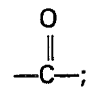

Z ist $—(CH_2)_m—$, wobei m 0 bis 2 ist,

mit der Maßgabe, daß nicht gleichzeitig m 0 ist und W abwesend ist; und

$R^6$ ist Wasserstoff; nieder-Alkyl; Halogen; oder $OR^4$;

$R^2$ ist $—(CH_2)_r—B—(CH_2)_s—NR^7R^{15}$

wobei

r und s unabhängig voneinander 0 bis 3 sind;

B ist abwesend; —O—; —S—; oder $—NR^8—$;

wobei $R^8$ Wasserstoff, nieder-Alkyl; Alkanoyl; oder Aroyl ist; und

$R^7$ ist

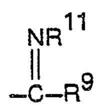 ; 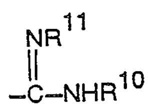 ; oder 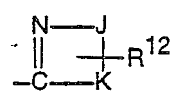

worin

$R^9$ ist nieder-Alkyl; Aralkyl; Aryl, Heteroaryl; oder Heteroar-nieder-alkyl und diese Gruppen substituiert durch Hydroxyl, nieder-Alkoxy oder Halogen; Carboxyl; Carboxamido; Nitromethenyl;

$R^{10}$ ist Wasserstoff; nieder-Alkyl; Aryl; oder Amidino;

$R^{11}$ ist Wasserstoff; nieder-Alkyl; Cyano; Amidino; Aryl; Aroyl; nieder-Alkanoyl;

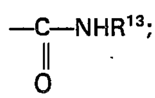

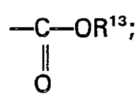

$—NO_2$; $—SO_2NH_2$; oder $SO_2R^{13}$;

$R^{12}$ ist Wasserstoff; nieder-Alkyl; Halogen; Aralkyl; Amino; Cyano; Mono- oder Di-nieder-alkylamino; oder $OR^4$;

$R^{13}$ ist Wasserstoff; nieder-Alkyl; oder Aryl;

$R^{15}$ ist Wasserstoff; nieder-Alkyl; Aralkyl; oder Aryl;

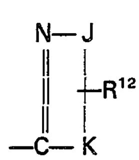

stellt einen basischen Heterocyclus mit 5 oder 6 Atomen oder benzokondensierte Analoge davon, die ggf. 1 bis 3 N-Atome, eine Sauerstoff-, eine Schwefel-, eine S=O— oder eine $SO_2$-Gruppe enthalten und ggf. durch Amino-, nieder-Alkylamino-, Di-nieder-alkylamino-, nieder-Alkoxy- oder Aralkylgruppen substituiert sein können, dar;

$R^3$ ist $C_3$—$C_8$-Cycloalkyl und benzokondensiertes $C_3$—$C_8$-Cycloalkyl; perhydrobenzokondensiertes $C_3$—$C_8$-Cycloalkyl; Aryl; substituiertes Aryl; Heteroaryl; substituiertes Heteroaryl;

$R^{14}$ ist Wasserstoff oder nieder-Alkyl; und pharmazeutisch verträgliche Salze davon.

4. Pharmazeutische Zusammensetzung zur Behandlung von Hochdruck, enthaltend einen pharmazeutisch verträglichen Trägerstoff; eine antihypertensiv wirksame Menge einer Verbindung nach Anspruch 1; und eine antihypertensive und/oder diuretische Verbindung, ausgewählt aus der Gruppe Amilorid, Atenolol, Bendroflumethiazid, Chlorothalidon, Chlorothiazid, Clonidin, Cryptenaminacetate und Cryptenamin-tannate, Deserpidin, Diazoxid, Guanethiden-sulfat, Hydralazin-hydrochlorid, Hydrochlorothiazid, Hydroflumethiazid, Indacrinon, Metolazon, Metoprolol-tartrat, Methylclothiazid, Methyldopa, Methyldopat-hydrochlorid, Minoxidil, Pargylin-hydrochlorid, Polythiazid, Prazosin, Propranolol, Rauwolfia serpentina, Rescinnamin, Reserpin, Natriumnitroprussid, Spironolacton, Timolol, Trichlormethiazid, Trimethophan-camsylat, Benthiazid, Quinetazon, Ticrynafen, Triamteren, Acetazolamid, Aminophyllin, Cyclothiazid, Äthacrynsäure, Furosemid, Merethoxyllin-procain und Natriumäthacrynat.

5. Eine Verfahren zur Herstellung der Verbindungen der Formeln I und Ia nach Anspruch 1, umfassend die Derivatisierung mit geeigneten Reagentien von substituierten N-Carboxymethyldipeptidin der Formeln

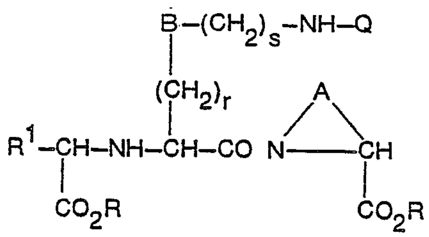

und

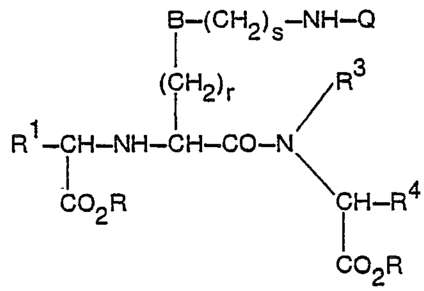

worin Q H oder

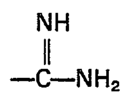

ist und R, $R^1$, B, r, s, $R^3$ und $R^4$ die in Anspruch 1 definierte Bedeutung haben, unter Bildung von Verbindungen der Formel I und Ia und ggf. Entfernung von eventuell vorhandenen Schutzgruppen nach Standardverfahren und ferner ggf. Herstellung der Salze dieser Verbindungen nach herkömmlichen Verfahren.

**Revendications**

1. Composés de formules:

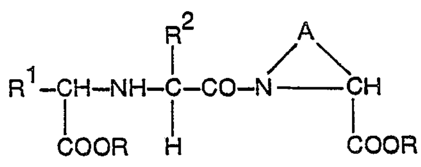

(I)

et

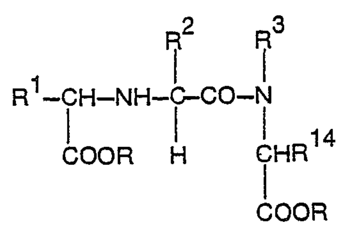

(Ia)

dans lesquelles:

R est un atome d'hydrogène; un radical alkyle inférieur; aralkyle; ou aryle;

$R^1$ représente un atome d'hydrogène; un radical alkyle en $C_{1-12}$ à chaîne ramifiée ou droite ou alcényle; un radical cycloalkyle en $C_{3-9}$ ou alkylbenzocondensé; un radical alkyle inférieur substitué dont les substituants sont halo, hydroxy, alcoxy inférieur, aryloxy, amino, mono- ou di-alkyl(inf)amino, acylamino,

arylamino, guanidino, mercapto, alkyl(inf)thio, arylthio, carboxy, carboxamido ou alcoxy(inf)carbonyle, aryle, aryle substitué dont les substituants sont alkyle inférieur; alcoxy inférieur, ou halo; aralkyle inferieur; aralcényle inférieur; hétéroaralkyle inférieur; hétéroaralcényle inférieur; aralkyle inférieur substitué, aralcényle inférieur substitué; hétéroaralkyle inférieur substitué ou hétéroaralcényle substitué dont les substituants aryle et hétéroaryle sont halo, dihalo, alkyle inférieur, hydroxy, alcoxy inférieur, amino, aminoalkyle inférieur, acylamino, mono- ou di-alkyl(inf)amino, carboxyle, haloalkyle inférieur, nitro, cyano ou sulfonamido, et dans lesquels la portion alkyle inférieur du radical aralkyle inférieur peut être substituée par amino, acylamino ou hydroxyle;

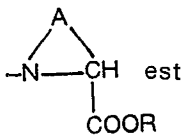 est 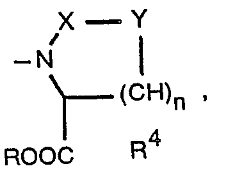 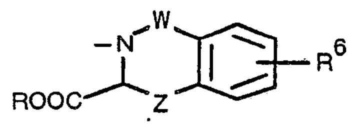

ou

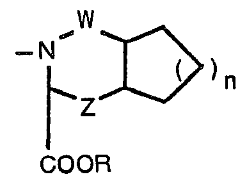

dans laquelle

X et Y représentent conjointement

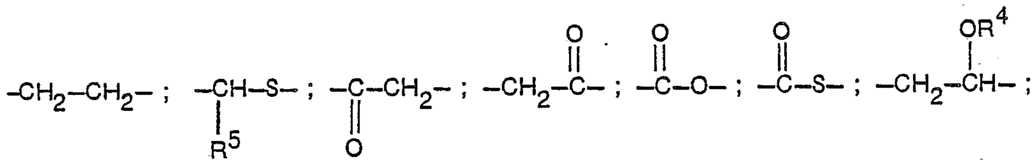

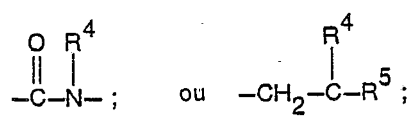

$R^4$ est un atome d'hydrogène; un radical alkyle inférieur; aryle; aryle substitué;

$R^5$ représente un atome d'hydrogène; un radical alkyle inférieur; aryle ou aryle substitué;

*n* est 1 à 3;

W est absent; $-CH_2-$ ou

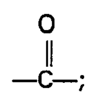

Z représente $-(CH_2)_m-$, dans lequel *m* est 0 à 2, à la condition que *m* ne puisse pas être 0 et W ne puisse pas être absent en même temps; et

$R^6$ représente un atome d'hydrogène; un radical alkyle inférieur; halo; ou $OR^4$;

$R^2$ est $-(CH_2)_r-B-(CH_2)_s-NR^7R^{15}$

dans laquelle

*r* et *s* représentent indépendamment 0 à 3;

B est absent; —O—; —S—; ou $-NR^8-$;

dans laquelle $R^8$ est l'hydrogène; alkyle inférieur; alcanoyle; ou aroyle; et

$R^7$ est

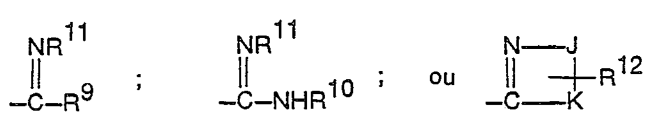

dans lequel

$R^9$ représente un radical alkyle inférieur; aralkyle; aryle; hétéroaryle; ou hétéroaralkyle inférieur; et les groupes substitués par hydroxy, alcoxy inférieur ou halo; carboxyle; carboxamido; nitrométhényle;

$R^{10}$ est un atome d'hydrogène; un radical alkyle inférieur; aryle; ou amidino;

$R^{11}$ représente un atome d'hydrogène; un radical alkyle inférieur; cyano; amidino; aryle; aroyle; alcanoyle inférieur;

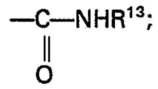

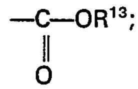

$—NO_2$; $—SO_2NH_2$; ou $SO_2R^{13}$;

$R^{12}$ est l'hydrogène; alkyle inférieur; halo; aralkyle; amino; cyano; mono- ou di-alkyl(inf)amino; ou $OR^4$;

$R^{13}$ est l'hydrogène; alkyl(inf); ou aryle;

$R^{15}$ est l'hydrogène; alkyl(inf); aralkyle; ou aryle;

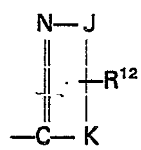

constitue un hétérocycle basique de 5 ou 6 atomes ou ses analogues benzocondensés et contient facultativement 1 à 3 atomes de N, un atome d'oxygène, un atome de soufre, un groupe S=O ou un groupe $SO_2$ facultativement substitué par amino, alkyl(inf)amino, dialkyl(inf)amino, alcoxy inférieur ou aralkyle;

$R^3$ est un radical cycloalkyle en $C_{3-8}$ ou cycloalkyle en $C_{3-8}$ benzocondensé; cycloalkyle en $C_{3-8}$ perhydrobenzocondensé; aryle; aryle substitué; hétéroaryle; hétéroaryle substitué;

$R^{14}$ est un atome d'hydrogène ou un radical alkyle inférieur; et leurs sels pharmaceutiquement acceptables.

2. Composé selon la revendication 1, qui est choisi dans le groupe des:
$N^2$-[1-(S)-carboxy-3-phénylpropyl]-$N^6$-(4,5-dihydro-2-thiazolyl)-L-lysyl-L-proline;
$N^2$-[1-(S)-carboxy-3-phénylpropyl]-$N^6$-(1-iminophényl)-L-lysyl-L-proline;
$N^2$-[1-(S)-carboxy-3-phénylpropyl]-nitro-L-arginyl-L-proline;
$N^2$-[1-(S)-éthoxycarbonyl-3-phénylpropyl]-$N^6$-[(cyanoamino)-iminométhyl]-L-lysyl-L-proline;
$N^2$-[1-(S)-carboxy-3-phénylpropyl]-$N^6$-[[(aminoiminométhyl)amino]iminométhyl]-L-lysyl-L-proline;
$N^2$-[1-(S)-éthoxycarbonyl-3-phénylpropyl]-$N^6$-(2-pyrimidinyl)-L-lysyl-L-proline;
$N^2$-[1-(S)-éthoxycarbonyl-3-phénylpropyl]-$N^5$-(2-benzothiazolyl)-L-ornithyl-L-proline; et
$N^2$-[1-(S)-carboxy-3-phénylpropyl]-carbamoyl-L-arginyl-L-proline.

3. Composition pharmaceutique utile pour traiter l'hypertension et comprenant un véhicle pharmaceutiquement acceptable et une dose efficace comme agent antihypertenseur d'un composé répondant aux formules:

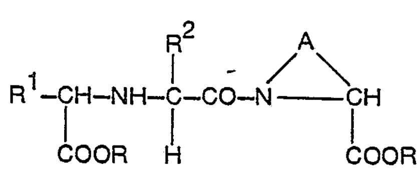

(I)

et

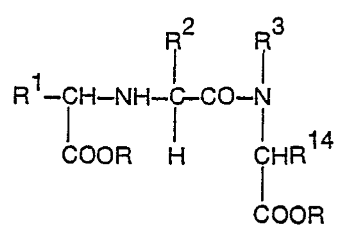

(Ia)

dans lesquelles:

R est un atome d'hydrogène; un radical alkyle inférieur; aralkyle; ou aryle;

$R^1$ représente un atome d'hydrogène; un radical alkyle en $C_{1-12}$ à chaîne ramifiée ou droite ou alcényle; un radical cycloalkyle en $C_{3-9}$ ou alkylbenzocondensé; un radical alkyle inférieur substitué dont les substituants sont halo, hydroxy, alcoxy inférieur, aryloxy, amino, mono- ou di-alkyl(inf)amino, acylamino, arylamino, guanidino, mercapto, alkyl(inf)thio, arylthio, carboxy, carboxamido ou alcoxy(inf)carbonyle,

aryle, aryle substitué dont les substituants sont alkyle inférieur, alcoxy inférieur, ou halo; aralkyle inférieur; aralcényle inférieur; aralcényle inférieur; hétéroaralkyle inférieur; hétéroaralcényle inférieur; aralkyle inférieur substitué, aralcényle inférieur substitué; hétéroaralkyle inférieur substitué ou hétéroaralcényle substitué dont les substituants aryle et hétéroaralyle sont halo, dihalo, alkyle inférieur, hydroxy, alcoxy inférieur, amino, aminoalkyle inférieur, acylamino, mono- ou di-alkyl(inf)amino, carboxyle, haloalkyle inférieur, nitro, cyano ou sulfonamido, et dans lesquels la portion alkyle inférieur du radical aralkyle inférieur peut être substituée par amino, acylamino ou hydroxyle;

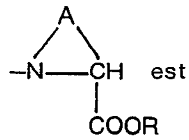 est 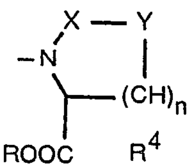 , 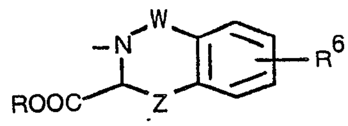

ou

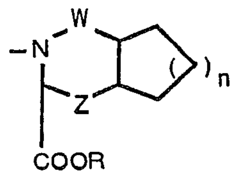

dans laquelle

X et Y représentent conjointement

$-CH_2-CH_2-$ ; 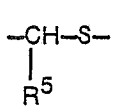 ; 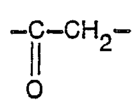 ; 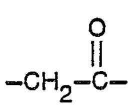 ; 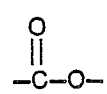 ; 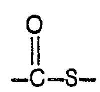 ; 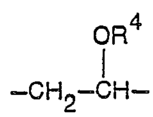 ;

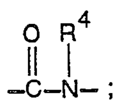 ; ou 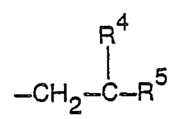 ;

$R^4$ est un atome d'hydrogène; un radical alkyle inférieur; aryle; aryle substitué;

$R^5$ représente un atome d'hydrogène; un radical alkyle inférieur; aryle ou aryle substitué;

*n* est 1 à 3;

W est absent; $-CH_2-$ ou

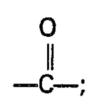;

Z represente $-(CH_2)_m-$, dans lequel *m* est 0 à 2, à la condition que *m* ne puisse pas être 0 et W ne puisse pas être absent en même temps; et

$R^6$ représente un atome d'hydrogène; un radical alkyle inférieur; halo; ou $OR^4$;

$R^2$ est $-(CH_2)_r-B-(CH_2)_s-NR'R''$

dans laquelle

*r* et *s* représentent indépendamment 0 à 3;

B est absent; —O—; —S—; ou —$NR^8$—;
dans laquelle $R^8$ est l'hydrogène; alkyle inférieur; alcanoyle; ou aroyle; et
$R^7$ est

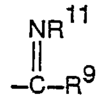 ; 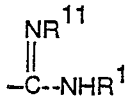 ; ou 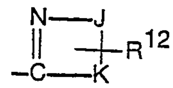

dans lequel

$R^9$ représente un radical alkyle inférieur; aralkyle; aryle; hétéroaryle; ou hétéroaralkyle inférieur; et les groupes substitués par hydroxy, alcoxy inférieur ou halo; carboxyle; carboxamido; nitrométhényle;

$R^{10}$ est un atome d'hydrogène; un radical alkyle inférieur; aryle; ou amidino;

$R^{11}$ représente un atome d'hydrogène; un radical alkyle inférieur; cyano; amidino; aryle; aroyle; alcanoyle inférieur;

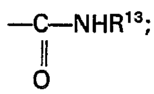

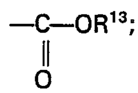

—$NO_2$; —$SO_2NH_2$; ou $SO_2R^{13}$;

$R^{12}$ est l'hydrogène; alkyle inférieur; halo; aralkyle; amino; cyano; mono- ou di-alkyl(inf)amino; ou $OR^4$;

$R^{13}$ est l'hydrogène; alkyl(inf); ou aryle;

$R^{15}$ est l'hydrogène; alkyl(inf); aralkyle; ou aryle;

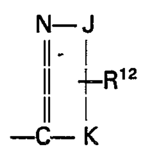

constitue un hétérocycle basique de 5 ou 6 atomes ou ses analogues benzocondensés et contient facultativement 1 à 3 atomes de N, un atome d'oxygène, un atome de soufre, un groupe S=O ou un groupe $SO_2$ facultativement substitué par amino, alkyl(inf)amino, dialkyl(inf)amino, alcoxy inférieur ou aralkyle;

$R^3$ est un radical cycloalkyle en $C_{3-8}$ ou cycloalkyle en $C_{3-8}$ benzocondensé; cycloalkyle en $C_{3-8}$ perhydrobenzocondensé; aryle; aryle substitué; hétéroaryle; hétéroaryle substitué;

$R^{14}$ est un atome d'hydrogène ou un radical alkyle inférieur; et leurs sels pharmaceutiquement acceptables.

4. Composition pharmaceutique utile pour le traitement de l'hypertension qui comprend un véhicule pharmaceutiquement acceptable, une proportion efficace come agent anti-hypertenseur d'un composé selon la revendication 1; et un composé anti-hypertenseur et/ou diurétique choisi dans le groupe comprenant les: amiloride, aténolol, bendrofluméthiazide, chlorothalidone, chlorothiazide, clonidine, acétates de crypténamine et tannates de crypténamine, déserpidine, diazoxyde, sulfate de guanéthidène, chlorhydrate d'hydralazine, hydrochlorothiazaide, hydrofluméthiazide, indacrinone, métolazone, tartrate de métoprolol, méthylclothiazide, méthyldopa, chlorhydrate de méthyldopate, minoxidile, chlorhydrate de pargyline, polythiazide, prazosine, propanolol, *rauwolfia serpentina,* rescinnamine, réserpine, nitroprusside de sodium, spironolactone, timolol, trichlorméthiazide, camsylate de triméthophane, benzothiazide, quinéthazone, ticrynafène, triamterène, acétozolamide, aminophylline, cyclothiazide, acide éthacrynique, furosémide, maréthoxylline-procaïne, et éthacrynate de sodium.

5. Procédé de préparation des composés de formules I et Ia selon la revendication 1, qui consiste à utiliser des réactifs appropriés de N-carboxyméthyldipeptides substitués pour la dérivation, composés qui répondent aux formules:

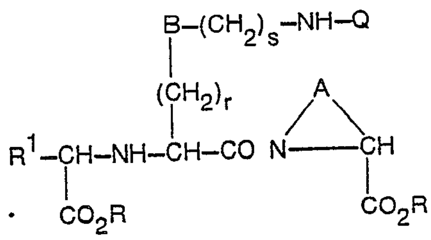

et

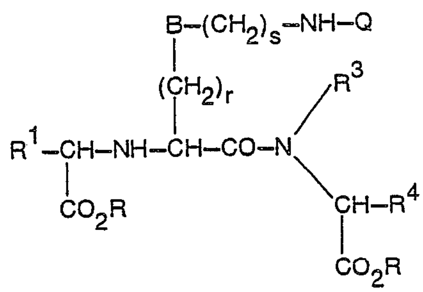

dans lesquelles Q est H ou

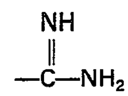

et R, $R^1$, B, r, s, $R^3$ et $R^4$ sont tels que définis dans la revendication 1 pour obtenir des composés de formules I et Ia et, éventuellement, éliminer les groupes protecteurs présents par des procédés normalisés et, également si on le désire, préparer des sels desdits composés par des moyens classiques.